(19) Europäisches Patentamt
European Patent Office
Office européen des brevets

(11) Publication number: **0 028 063**
**B1**

(12) **EUROPEAN PATENT SPECIFICATION**

(45) Date of publication of patent specification: **13.06.84**

(21) Application number: **80303101.2**

(22) Date of filing: **04.09.80**

(51) Int. Cl.[3]: **C 07 C 65/32,**
C 07 C 59/84,
C 07 C 79/46,
C 07 C 79/38,
C 07 D 257/04,
C 07 D 257/06,
A 61 K 31/04,
A 61 K 31/12,
A 61 K 31/19,
A 61 K 31/21, A 61 K 31/40

(54) **Phenol derivatives, processes for their preparation and pharmaceutical compositions containing them.**

(30) Priority: **05.09.79 GB 7930849**

(43) Date of publication of application:
**06.05.81 Bulletin 81/18**

(45) Publication of the grant of the patent:
**13.06.84 Bulletin 84/24**

(84) Designated Contracting States:
**AT BE CH DE FR IT LI NL SE**

(56) References cited:
**DE - A - 2 749 518**
**GB - A - 1 144 906**
**GB - A - 1 405 254**

(73) Proprietor: **GLAXO GROUP LIMITED**
**Clarges House 6-12 Clarges Street**
**London W1Y 8DH (GB)**

(72) Inventor: **Oxford, Alexander William**
**60 Green Drift**
**Royston Hertfordshire (GB)**
Inventor: **Ellis, Frank**
**36 Blandford Avenue**
**Luton Bedfordshire (GB)**

(74) Representative: **Kyle, Diana et al,**
**ELKINGTON AND FIFE High Holborn House 52/54**
**High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 028 063 B1

**Description**

This invention relates to phenol derivatives, to processes for their preparation and to pharmaceutical compositions containing them.

More particularly, this invention relates to phenoxyalkoxyphenyl derivatives and processes for their preparation.

Reference is made to U.K. Patent Specification No. 1,405,254 which describes and claims certain bis-(substituted-phenoxy) propanes of formula

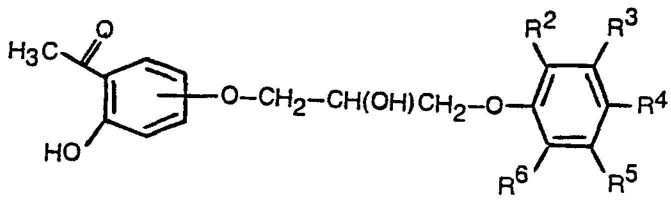

These compounds carry only two substituents (an acetyl group and a hydroxy group) on the phenoxy part of the molecule. The compounds are described as intermediates to biologically active compounds.

Reference is also made to U.K. Patent Specification No. 1,144,906 which describes and claims bis phenoxy derivatives. The compounds are described as intermediates in the preparation of chromone derivatives.

The present invention provides a compound of the general formula (I)

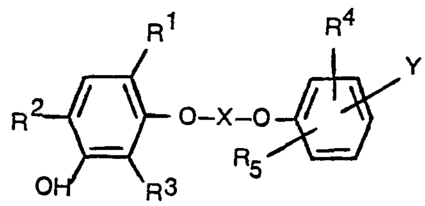

(I)

and physiologically acceptable salts thereof.

In the general formula (I),

Y represents the group —A, —ZA or $OZ^1A$ where A represents a carboxylic acid group, a 5-1H-tetrazolyl ring, or N-5-1H-tetrazolylcarboxamide group;

Z represents a $C_{1-4}$ alkylene, $C_{2-4}$ alkenylene or $C_{2-4}$ alkynylene chain optionally substituted by one or more $C_{1-3}$ alkyl groups and $Z^1$ represents a $C_{1-4}$ alkylene chain optionally substituted by one or more $C_{1-3}$ alkyl groups;

X represents a $C_{1-10}$ carbon chain which may be saturated or unsaturated in the case of chains containing at least 4 carbon atoms, and may be substituted by a hydroxy group or by one or more $C_{1-3}$ alkyl groups, which chain may be interrupted by a benzene ring which may be linked through its 1- and 2-, 1- and 3-, or 1- and 4-positions;

$R^1$ represents a hydrogen atom or a $C_{1-6}$ alkyl group;

$R^2$ represents the group —$COR^6$ where $R^6$ represents a hydrogen atom, an aryl group or a $C_{1-6}$ alkyl group which may be substituted by an aryl group;

$R^3$ represents a $C_{1-6}$ alkyl group or a $C_{3-6}$ alkenyl group; and

$R^4$ and $R^5$, which may be the same or different, each represents a hydrogen atom, a halogen atom or a hydroxy, $C_{1-3}$ alkoxy, $C_{1-6}$ alkyl, $C_{3-6}$ alkenyl, $C_{1-3}$ alkanoyl, nitro or carboxylic acid group, with the proviso that $R^4$ and $R^5$ cannot both be nitro, alkanoyl or carboxylic acid groups.

Referring to the general formula (I), the alkyl group represented by $R^1$, $R^2$, $R^3$, $R^4$ and $R^5$ and the alkenyl groups represented by $R^3$, $R^4$ and $R^5$ may be a straight chain or branched chain group.

When Y is the group A, A preferably represents a carboxylic acid group or a 5-1H-tetrazolyl ring.

When Y is the group ZA or $OZ^1A$, A preferably represents a carboxylic acid group, Z preferably represents a $C_{1-4}$ alkylene chain, particularly methylene or ethylene, or a $C_{2-4}$ alkenylene chain, particularly ethenylene, or a $C_{2-4}$ alkynylene chain, particularly ethynylene, and $Z^1$ preferably represents methylene.

Examples of the group X are a straight carbon chain of formula $(CH_2)_n$ where *n* is a number from 1 to 10, or a group of formula —$CH_2X^1CH_2$— where $X^1$ is a benzene ring linked through its 1- and 2-, 1- and 3-, or 1- and 4-positions. X preferably represents a group of formula $(CH_2)_n$ where *n* is 1,3 or 5.

$R^1$ preferably represents a hydrogen atom or a $C_{3-6}$ alkyl group, particularly propyl.

$R^2$ is preferably the group $COR^6$ where $R^6$ represents a $C_{1-6}$ alkyl group, particularly a methyl group. When $R^6$ is an aryl group it is, for example, a phenyl group.

$R^3$ preferably represents a $C_{3-6}$ alkyl group, particularly propyl, or $C_{3-6}$ alkenyl group, particularly propenyl.

$R^4$ preferably represents a hydrogen or halogen atom, or a hydroxy, nitro or $C_{1-3}$ alkanoyl group. When $R^4$ is a halogen atom it is preferably bromine. The $C_{1-3}$ alkanoyl group represented by $R^4$ is preferably acetyl.

$R^5$ is preferably a hydrogen atom or a $C_{1-6}$ alkyl group, particularly propyl, or a $C_{2-4}$ alkenyl group, particularly propenyl.

Particularly preferred compounds are those in which $R^6$ is a hydrogen atom or a hydroxyl group and $R^5$ is a hydrogen atom.

A preferred group of compounds are those of formula (II):—

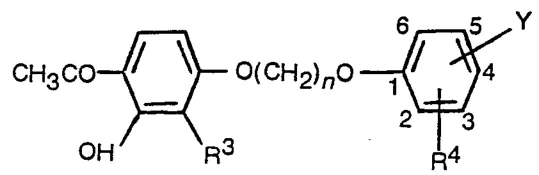

(II)

where Y is a carboxylic acid group or a 5-1H-tetrazolyl ring or a N-5-1H-tetrazolylcarboxamide group, or a $C_{1-2}$ alkyl, $C_{1-2}$ alkoxy or $C_2$-alkenyl chain terminally substituted by a carboxylic acid group, and is preferably situated at the 3- or 4-position in the benzene ring;
*n* is 1, 3 or 5, preferably 3,
$R^3$ is a $C_{3-6}$ alkyl or $C_{3-6}$ alkenyl group,
$R^4$ is a hydrogen or bromine atom, or a hydroxy, nitro or acetyl group,
and physiologically acceptable salts thereof.

In formula (II), when $R^4$ is a hydroxy group it is preferably situated adjacent to the group Y.

Preferred salts of the above acids include salts obtained by reaction with an inorganic or organic base, for example sodium salts or salts with ethylenediamine.

Particularly preferred compounds according to the invention include:
4-[3-(4-Acetyl-3-hydroxy-2-propylphenoxy)propoxy]-2-hydroxybenzoic acid;
3-[3-(4-Acetyl-3-hydroxy-2-propylphenoxy)propoxy]benzoic acid;
3-[4-[3-(4-Acetyl-3-hydroxy-2-propylphenoxy)-propoxy]phenyl]-2-propenoic acid;
4-[3-(4-Acetyl-3-hydroxy-2-propylphenoxy)propoxy]benzoic acid;
3-[3-(4-Acetyl-3-hydroxy-2-propylphenoxy)propoxy]benzenepropanoic acid;
2-[4-[3-(4-Acetyl-3-hydroxy-2-propylphenoxy)propoxy]phenoxy]acetic acid;
3-[3-[3-(4-Acetyl-3-hydroxy-2-propylphenoxy)propoxy]phenyl]-2-propenoic acid;
4-[3-(4-Acetyl-3-hydroxy-2-propylphenoxy)propoxy]-2-hydroxy-3-propylbenzoic acid;
3-[5-[3-(4-Acetyl-3-hydroxy-2-propylphenoxy)propoxy]-2-bromophenyl]-2-propenoic acid;
3-[3-[5-(4-Acetyl-3-hydroxy-2-propylphenoxy)pentyloxy]phenyl]-2-propenoic acid;
3-[3-[3-[4-Acetyl-3-hydroxy-2-propylphenoxy]propoxy]-4-nitrophenyl]-2-propenoic acid;
3-[3-[3-[4-Acetyl-3-hydroxy-2-propylphenoxy]propoxy]-4-acetylphenyl]-2-propenoic acid;
5-[3-[3-[4-Acetyl-3-hydroxy-2-propylphenoxy]propoxy]phenyl]-1H-tetrazole;
3-[3-[3-[4-Acetyl-3-hydroxy-2-[2-propenyl]phenoxy]propoxy]phenyl]-2-propenoic acid;
3-[3-[3-[4-Acetyl-3-hydroxy-2-(1-methylpropyl)phenoxy]propoxy]phenyl]-2-propenoic acid;
and the physiologically acceptable salts of these compounds.

The invention also extends to all possible enantiomers and diasteriomers of the compounds according to the formula (I).

The compounds according to the invention are potent antagonists of the action of slow reacting substance of anaphylaxis (SRS-A) as shown by their ability to inhibit the SRS-A induced contraction of guinea pig ileum (Coleman *et al*, Br. J. Pharmacol., 1979, *66*, 83P). SRS-A is a naturally occurring substance which causes contraction of smooth muscle, especially bronchial muscle, and increases vascular permeability. The substance is not normally present in human tissue but is synthesised and released from certain cells during an allergic reaction. The compounds according to the invention are thus indicated for use in the treatment of disorders in which SRS-A may be a factor, for example, in obstructive airways diseases such as asthma, in hay fever and in skin afflications.

The compounds according to the invention may be formulated for use in human or veterinary medicine for therapeutic or prophylactic purposes. The invention therefore includes within its scope pharmaceutical compositions comprising as active ingredients one or more compounds selected from compounds of general formula (I) and physiologically acceptable addition salts thereof. Such compositions may be presented for use in a conventional manner which the aid of physiologically acceptable carriers or excipients and formulatory agents as required, and with or without supplementary medicinal agents. These compositions include, for instance, tablets, suppositories,

injections and forms suitable for administration by inhalation. Injections may be formulated with the aid of physiologically acceptable carriers and agents as solutions, suspensions or as dry products for reconstitution before use.

For administration by inhalation the compounds are conveniently delivered in the form of an aerosol spray presentation from pressurised packs or a nebuliser. The preferred composition for inhalation is a powder which may be formulated as a cartridge from which the powdered composition may be inhaled with the aid of a suitable device. In the case of a pressurised aerosol the dosage unit may be determined by providing a valve to deliver a metered amount.

The doses of the active ingredient which may be used vary within a wide range. Suitable dosage units are generally within the range of 50 mg to 1000 mg/day which may be conveniently administered in two or three daily doses. The precise dose administered will always depend on the age and condition of the patient.

The compounds according to the invention may be prepared by a number of processes. According to one embodiment of the invention compounds of formula (I) may be prepared from the corresponding compounds of general formula (III):

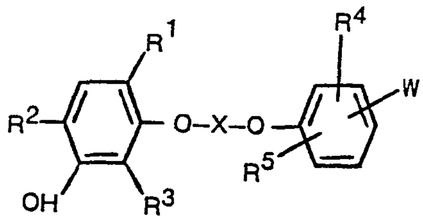

(III)

in which

$R^1$ to $R^5$ and X are as previously defined and W represents —CHO, —E, —ZE or $OZ^1E$ in which Z and $Z^1$ are as previously defined and E represents a group which is convertible to the group A previously defined, such as carboxylic ester, carboxamide or nitrile group.

Thus, for example, a compound of the general formula (I) in which —Y represents the group —$CH{=}CHCO_2H$ may be prepared from the corresponding compound of general formula (III) in which W represents —CHO, by the Perkin synthesis of cinnamic acids, as described in Organic Reactions, John Wiley & Sons, New York, Vol I, pp 210—265.

A compound of general formula (I), in which Y represents the group —A, —ZA or —$OZ^1A$ where Z and $Z^1$ are as previously defined and A is a carboxylic acid group, may be prepared by acid or base hydrolysis of the corresponding compound of general formula (III) in which W represents the group —E, —ZE or —$OZ^1E$ where E is a carboxylic ester, amide or nitrile group using standard conditions for example by hydrolysis with an acid such as aqueous sulphuric acid at an elevated temperature, or by hydrolysis with a base such as sodium hydroxide, potassium hydroxide or lithium hydroxide in a solvent such as ethanol, aqueous tetrahydrofuran or water at a temperature of 20—100°C.

A compound of general formula (I), in which Y represents the group —A, —ZA or $OZ^1A$ where Z and $Z^1$ are as previously defined and A is a 5-1H-tetrazolyl ring, may be prepared from the corresponding compound of general formula (III) in which W represents —E, —ZE or —$OZ^1E$ and E represents a nitrile, using standard conditions, for example using a salt of hydrazoic acid in a solvent such as dimethylformamide or dioxan at an elevated temperature. Suitable salts include sodium or ammonium azide. The reaction may be performed, for example, using sodium azide and ammonium chloride in dimethylformamide at a temperature of 50 to 100°C.

A compound of general formula (I) in which Y represents the group —A, —ZA or —$OZ^1A$ where Z and $Z^1$ are as previously defined and A is a carboxylic acid group, may be prepared by hydrogenolysis of the corresponding compound of general formula (III) where W represents the group —E, —ZE or —$OZ^1E$ where E is a carboxylic ester group of formula $COOR^7$ and $R^7$ is a group which may be removed by hydrogenolysis, for example an arylmethyl group such as a benzyl or benzhydryl group, using standard conditions, for example in the presence of a catalyst, such as platinum or palladium in a suitable solvent such as an alcohol e.g. ethanol.

This reaction may be conveniently employed concurrently to reduce other groups in a compound of general formula (III). For example, a compound of general formula (I) in which A is a carboxylic acid group and $R_3$ is a $C_{2-6}$ alkyl group, such as propyl, may be prepared by catalytic hydrogenation of the corresponding compound of general formula (III) where W represents the group E, ZE or $OZ^1E$ where Z and $Z^1$ are as previously defined and E is a carboxylic ester group, such as a benzyl ester group, and $R^3$ is a $C_{2-6}$ alkenyl group, such as propenyl.

The intermediate compounds of formula (III) may be prepared by a number of processes. For example, the compounds of formula (III) may be obtained by alkylation of the corresponding phenols of general formula (IV) or (V):

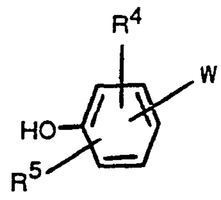

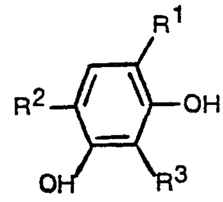

(IV) (V)

with an appropriate alkylating agent of formula (VI) or (VII) respectively,

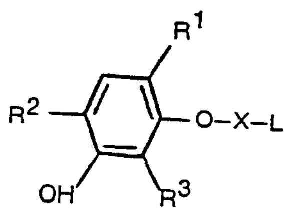

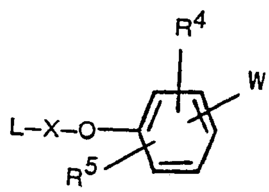

(VI) (VII)

in which

L is a leaving group for example a halogen atom or a paratoluenesulphonate or a methylsulphonate ester,

in the presence of a base in a suitable solvent, for example sodium hydride in dimethylformamide or potassium carbonate in butanone.

The alkylating agents of formula (VI) or (VII) may be prepared from the corresponding phenols of formula (V) or (IV) respectively by treatment with a compound L—X—$L^1$ (where L is a leaving group as previously described and $L^1$ is the same or different leaving group, or is a group such as hydroxy group which may be converted into a leaving group by conventional methods) in the presence of a base in a suitable solvent as described above for the preparation of compounds of formula (III).

The phenols of formulae (IV) and (V) are either known compounds or may be prepared from known starting materials by methods analogous to those used for the preparation of the similar known phenols.

For example, phenols of formula (IV) in which W represents the group —E, ZE or $OZ^1E$ where Z and $Z^1$ are as previously described and E is a carboxylic ester group may be prepared using methods similar to that described by Carter and Lawrence, J. Chem. Soc. *77*, 1226 (1900), or by esterification of the corresponding acids using standard conditions. The acids are either known or may be prepared from known starting materials by conventional methods for example by the Perkin synthesis as previously described for the preparation of compounds of formula (I).

Phenols of formula (V) in which $R^1$ is a hydrogen atom may, for example, be prepared according to the methods of R. A. Appleton *et al* J. Med. Chem., *20* 371 (1977) or W. Baker and O. M. Lothian in J. Chem. Soc., 628 (1935).

It will be appreciated that some of the intermediates of general formula (IV) and (VII) may, if desired, be modified by electrophilic substitution using standard conditions, for example by nitration with a nitrating agent, such as concentrated nitric acid at low temperatures, or by bromination with a brominating agent, such as N-bromoacetamide in a solvent such as dioxan at an elevated temperature.

A compound of general formula (III) in which the chain —X— is substituted by a hydroxy group may be obtained by reaction of a compound of general formula (VIII) or (IX),

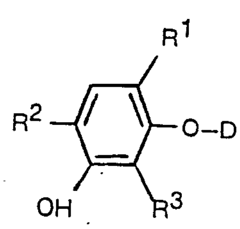

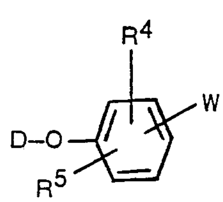

(VIII) (IX)

wherein D represents a hydrocarbon chain carrying an epoxide group, with a phenol of general formula (IV) or (V) respectively in a suitable solvent, for example dimethylformamide, in the presence of a catalyst such as trimethylbenzylammonium hydroxide at an elevated temperature.

An epoxide of formula (VIII) or (IX) may be obtained from a phenol of formula (V) or (IV) respectively by treatment with an alkyl halide carrying an epoxide group, for example epichlorohydrin, in the presence of a base such as potassium carbonate and in a solvent such as butanone.

Certain of the compounds of general formula (I) may be prepared from other compounds of general formula (I). Thus for example compounds of formula (I) in which Y represents the group —ZA where Z is a $C_{2-4}$ alkylene or $C_{2-4}$ alkenylene chain may be prepared by catalytic hydrogenation of a corresponding compound of formula (I) where Z is a $C_{2-4}$ alkenylene or $C_{2-4}$ alkynylene chain. Conventional catalysts may be used, preferably palladium in a suitable solvent, for example an alcohol such as methanol.

A compound of general formula (I) in which Y represents the group —A, —ZA or $OZ^1A$ where A is a N-5-1H-tetrazolyl-carboxamide may be prepared from the corresponding compounds of formula (I) in which A represents a carboxylic acid group using standard conditions, for example using 5-aminotetrazole and a coupling agent such as N,N'-carbonyldiimidazole in a solvent such as dimethylformamide or tetrahydrofuran.

It will be appreciated that certain of the reactions described above are capable of affecting other groups in the starting material which are desired in the end products; care must therefore be taken, in accordance with conventional practice either to perform reactions in a sequence which does not modify groups which are to be retained in the end products, or to ensure that such groups are in protected forms before the start of the reaction.

This applies in particular to the preparation of the intermediates of formula (III) where it may be necessary to protect hydroxyl groups in starting materials (IV) and (VII) which are not involved in the alkylation reaction.

The hdyroxy groups may be protected as, for example, a benzyl ether or an acetate during the synthesis. The protecting group is removed when required either by catalytic hydrogenolysis or by hydrolysis as appropriate.

A physiologically acceptable salt of a compound of general formula (I) may be prepared by standard techniques for example by treatment of the free acid of formula (I) with a base such as sodium hydroxide.

The following Examples illustrate the invention. All temperatures are in °C and "petroleum ether" refers to petroleum ether (b.p. 60—80°C).

Preparation 1

2-Hydroxy-4-(2-propenyloxy)benzoic acid, methyl ester

Methyl 2,4-dihydroxybenzoate (112 g), allyl bromide (78.6 g), potassium carbonate (92 g) and sodium iodide (1 g) were heated under reflux in butanone (1120 ml) for $2\frac{1}{2}$ hours. The mixture was cooled and filtered and the filtrate evaporated to dryness. The residue was distilled under reduced pressure to give the *title compound* as an oil (114.5 g) with b.p. 92° at $8 \times 10^{-2}$ torr.

Preparation 2

2,4-Dihydroxy-3-(2-propenyl)benzoic acid, methyl ester

The compound of Preparation 1 (107.7 g) was heated at 200° in a nitrogen atmosphere for $2\frac{1}{2}$ hours. The oil was distilled under reduced pressure to give the *title ester* (89.5 g) as white crystals with b.p. 110° at $4 \times 10^{-2}$ torr and m.p. 56—58°.

Preparation 3

2,4-Dihydroxy-3-propylbenzoic acid, methyl ester

The compound of Preparation 2 (72 g) was hydrogenated at atmospheric pressure in ethanol (1050 ml) using 10% palladium oxide on charcoal (5 g) as catalyst. The mixture was filtered, and the filtrate evaporated to dryness and the residue distilled under reduced pressure to give the *title ester* (68.9 g) b.p. 118° at $7 \times 10^{-2}$ torr, m.p. 59—61°.

Preparation 4

(a) 1-[4-(4-Bromomethyl)phenylmethoxy-2-hydroxy-3-propylphenyl]ethanone

1-(2,4-Dihydroxy-3-propylphenyl)ethanone (5 g), 1,4-bis(bromomethyl)benzene (13.6 g) and potassium carbonate (7.5 g) were heated under reflux in butanone (100 ml) for 2 hours. The mixture was cooled, acidified with 2N hydrochloric acid and extracted with ether (50 ml). The organic phase was washed with saturated brine, dried and evaporated to dryness. The residue was partially purified by chromatography on silica using petroleum ether as eluent to remove the excess of reagent and ethyl acetate as eluent to remove the crude product. The crude product was purified further by chromatography on silica using a mixture of petroleum ether (b.p. 60—80°) and dichloromethane (3:7) as the eluent to give the *title compound* (3.3 g) with m.p. 97—98°.

(b) 1-[4-(4-Chloro-2-butynyloxy)-2-hydroxy-3-propylphenyl]ethanone, m.p. 57—58° (from petroleum ether (b.p. 60—80°)) was prepared by a similar procedure from 1-(2,4-dihydroxy-3-propylphenyl)ethanone and 1,4-dichloro-2-butyne.

Preparation 5

1-[2-Hydroxy-4-(3-iodopropoxy)-3-propylphenyl]ethanone

1-[4-(3-Chloropropoxy)-2-hydroxy-3-propylphenyl]ethanone (20 g) and sodium iodide (22 g) were heated under reflux in butanone (400 ml) for 68 hours. The mixture was filtered, diluted with ether (200 ml) and washed with water and saturated brine. The organic phase was dried and evaporated to give an oil which was dissolved in petroleum ether. The solution was filtered and evaporated to dryness to give a solid which was recrystallised from aqueous isopropanol to give the *title iodide* (24.1 g) with m.p. 53—54°C.

Preparation 6

(a) 3-[3-(3-Iodopropoxy)phenyl]-2-propenoic acid, methyl ester

3-(3-Hydroxyphenyl)-2-propenoic acid, methyl ester (15 g), 1-bromo-3-chloropropane (13.2 g) and potassium carbonate (15 g) were heated under reflux in butanone (150 ml) for 24 hours. The mixture was cooled and filtered and the filtrate heated under reflux with sodium iodide (19 g) for 7 hours. The mixture was cooled and filtered and the filtrate diluted with ethyl acetate (200 ml) and washed with water, dried and evaporated. The residual oil was purified by chromatography on silica using a mixture of petroleum ether and ethyl acetate (3:1) as eluent. The major product was crystallised from petroleum ether (b.p. 60—80°) to give the *title iodide* (26 g) with m.p. 41—42°.

(b) 3-[3-(5-Iodopentyloxy)phenyl]-2-propenoic acid, methyl ester, b.p. 150° at $1 \times 10^{-2}$ torr. was prepared by a similar procedure from 3-(3-hydroxyphenyl)-2-propenoic acid, methyl ester and 1,5-dibromopentane.

Preparation 7

(a) 1-[2-Hydroxy-4-(4-iodobutoxy)-3-propylphenyl]ethanone

A mixture of 1-(2,4-dihydroxy-3-propylphenyl)ethanone (5 g), 1,4-dibromobutane (12 g), potassium carbonate (18 g) and butanone (100 ml) was heated under reflux for 5 hours. The cooled mixture was acidified with 2N hydrochloric acid and extracted with ether. The organic phase was washed with 2N sodium hydroxide solution, dried and concentrated. The residual crude bromide was dissolved in acetone (150 ml) and heated under reflux for 1 hour with sodium iodide (20 g). The cooled mixture was diluted with ether (200 ml), filtered and the filtrate evaporated to dryness. The residue was purified by chromatography on silica using a mixture of petroleum ether and ethyl acetate (4:1) as the eluent to give the *title compound* as a viscous oil (6.2 g).

(b) 1-[2-Hydroxy-4-(2-iodoethoxy)-3-propylphenyl]ethanone (m.p. 68—70°, from petroleum ether) was prepared by a similar procedure from 1-(2,4-dihydroxy-3-propylphenyl)ethanone and 1-bromo-2-chloroethane.

Preparation 8

1-(2,4-Dihydroxy-3,5-dipropylphenyl)ethanone

1-[2,4-Dihydroxy-3,5-di(2-propenyl)phenyl]ethanone (2.1 g) was hydrogenated in ethanol (50 ml) using 10% palladium oxide on charcoal (0.1 g) as catalyst. The mixture was filtered and the filtrate was evaporated to dryness and the residue recrystallised from toluene to give the *title compound* (1.8 g) with m.p. 98—99°.

Preparation 9

3-[2-Bromo-5-hydroxyphenyl]-2-propenoic acid, methyl ester

Bromine (1.3 ml) in methanol (40 ml) was added dropwise to a boiling solution of 3-(3-hydroxyphenyl)-2-propenoic acid (4 g) in methanol (40 ml) illuminated with a 100 watt lamp. The addition was completed in 15 min, and the cooled solution was evaporated to dryness. The residue was crystallised from cyclohexane to give the *title compound* (2.9 g) with m.p. 128—30°.

Preparation 10

1-[2-Hydroxy-4-(3-hydroxypropoxy)-3-propylphenyl]ethanone

1-(2,4-Dihydroxy-3-propylphenyl)ethanone (100 g), chloropropanol (48.7 g), potassium carbonate (141 g) and sodium iodide (5 g) were heated under reflux in butanone (500 ml) for 76 h. The mixture was filtered and the filtrate diluted with ethyl acetate (1 L). The solution was washed with 2 N sodium hydroxide solution (3 × 250 ml) and 10% sodium thiosulphate solution (3 × 500 ml), dried and evaporated to give the crude *title compound* as an oil (109 g).

Preparation 11

1-[2-Hydroxy-4-[3-[[(4-methylphenyl)sulphonyl]oxy]propoxy]-3-propylphenyl]ethanone

The compound of Preparation 10 (39 g), pyridine (50 ml) and 4-toluenesulphonyl chloride (30 g)

were stirred in dry ether (250 ml) for 16 h. The mixture was diluted with ethyl acetate (600 ml) and washed with water (2 x 500 ml), 8% sodium bicarbonate solution (500 ml), and 2 N hydrochloric acid (2 x 500 ml), dried and evaporated to dryness. The residue was triturated with petroleum ether and recrystallised from a mixture of ethyl acetate and petroleum ether to give the *title compound* (38 g) with m.p. 88°C.

Preparation 12

1-[4-(2-Butenyloxy)-2-hydroxyphenyl]ethanone

3-Chloro-1-butene (20 g) and sodium iodide (34 g) were heated under reflux in butanone (200 ml) for 25 h. The mixture was filtered and the filtrate heated under reflux with 1-(2,4-dihydroxyphenyl)ethanone (33.6 g) and potassium carbonate (63 g) for 4 days. The cooled reaction mixture was filtered and the filtrate evaporated to dryness. The residue was extracted with petroleum ether (300 ml) and the solution evaporated to give an oil which was fractionally distilled at 162—175° at 0.1 torr. The resulting oil was purified by chromatography on silica using a mixture of petroleum ether and ethyl acetate (6:1) as the eluent. The fractions containing the component which had an Rf of 0.6 on t.l.c. (silica; petroleum ether: ethyl acetate 4:1) were collected and evaporated to dryness to give the *title compound* (20.1 g) as an oil.

Preparation 13

1-[2,4-Dihydroxy-3-[1-methyl-2-propenyl]phenyl]ethanone

The compound of Preparation 12 (1 g) was heated at 180—190° for 6 h under nitrogen. The solid which sublimed was collected and purified by chromatography on silica using a mixture of petroleum ether and ethyl acetate (5:1) as the eluent to give the *title compound* (0.4 g) with m.p. 146—147°.

Preparation 14

1-[2,4-Dihydroxy-3-(1-methylpropyl)phenyl]ethanone

The compound of Preparation 13 (0.4 g) was hydrogenated in ethanol (70 ml) using 5% palladium on charcoal as the catalyst. The mixture was filtered and the filtrate evaporated to dryness to give the *title compound* (0.4 g) with m.p. 174—175°.

Preparation 15

3-[4-Acetyl-3-hydroxyphenyl]-2-propenoic acid, methyl ester

Aluminium chloride (14.97 g) was added to a mixture of 3-[3-hydroxyphenyl]-2-propenoic acid, methyl ester (10 g) and acetyl chloride (8 ml) in 1,2-dichloroethane (150 ml) at 0°. The mixture was stirred at 23° for 2 h and heated under reflux for 168 h. The mixture was cooled, diluted with water and extracted with ethyl acetate. The extracts were dried and evaporated to dryness to give an oil which crystallised on the addition of petroleum ether. The solid was heated under reflux in a mixture of ethanol (50 ml) and 2 N sodium hydroxide (250 ml) for 1 h. The mixture was acidified with 2 N hydrochloric acid and extracted with ethyl acetate. The extracts were dried and evaporated and the residue recrystallised from toluene to give the crude acid.

The crude acid was heated under reflux in methanol (200 ml) containing concentrated sulphuric acid (1 ml) for 2 h. The mixture was concentrated to 50 ml, 8% sodium bicarbonate solution added and extracted with ethyl acetate. The extracts were dried and evaporated and the residue recrystallised twice from cyclohexane to give the *title ester* (0.66 g) with m.p. 108—110°.

Preparation 16

2-Hydroxy-4-(3-hydroxypropoxy)benzoic acid, methyl ester

2,4-Dihydroxybenzoic acid, methyl ester (70 g) bromopropanol (58 g) and potassium carbonate (57.5 g) were heated under reflux in butanone (1 l) for 28 h. The mixture was filtered, the filtrate was evaporated and the residue dissolved in ethyl acetate (500 ml). The solution was washed with 8% sodium bicarbonate solution, water and saturated brine, dried and evaporated. The residual crude alcohol was recrystallised from cyclohexane to give the *title compound* (56.6 g), with m.p. 76—77°.

Preparation 17

2-Hydroxy-4-[3-[[(4-methylphenyl)sulphonyl]oxy]propoxy] benzoic acid, methyl ester

The compound of Preparation 16 (100 g), pyridine (70 ml) and 4-toluenesulphonyl chloride (104 g) were stirred in ether (1500 ml) in an ice bath for 2 h and then at room temperature for 58 h. The reaction mixture was filtered and the filtrate was washed with 2 N hydrochloric acid, water and sodium bicarbonate, dried and evaporated to give an oil which solidified when triturated with ether. The crystals were washed with dilute hydrochloric acid, air dried and crystallised from a mixture of ethanol and water to give the *title compound* (102 g), with m.p. 111—112.5°.

Preparation 18

2,4-Dihydroxy-3-propylbenzoic acid

The compound of Preparation 3 (2 g) was heated at 100° for 1 h in 2 N sodium hydroxide (10 ml). The solution was cooled and acidified and the precipitate filtered off and recrystallised from water to give the *title acid* (0.6 g), with m.p. 176—178°.

Preparation 19

2-Propylbenzene-1,3-diol

The compound of Preparation 18 (130 g) was heated under nitrogen at 190° for 1.5 h. The mixture was cooled, dissolved in ether and the solution was washed with 4% sodium bicarbonate solution dried and evaporated. The residue was recrystallised from cyclohexane to give the *title diol* (59.6 g), with m.p. 97—99°.

Preparation 20

a) (2,4-Dihydroxy-3-propylphenyl)phenylmethanone

The compound of Preparation 19 (2 g), anhydrous zinc chloride (4 g) and benzoic acid (7.3 g) were heated at 150° for 2.5 h. The reaction mixture was cooled and dissolved in a mixture of 2 N hydrochloric acid and ethyl acetate. The layers were separated and the organic phase washed with 4% sodium bicarbonate (3 x 50 ml), dried and evaporated. The residue (3.5 g) was purified by chromatography on silica (150 g) using a mixture of petroleum ether and ethyl acetate (4:1) as the eluent and recrystallised from cyclohexane to give the *title ketone* (2.1 g), with m.p. 152—153°.

The following compounds were prepared by a similar procedure from the compound of Preparation 19 and the appropriate acid.

b) 1-(2,4-Dihydroxy-3-propylphenyl)-1-propanone m.p. 104—106° from propionic acid

c) 1-(2,4-Dihydroxy-3-propylphenyl)-2-phenylethanone, m.p. 128—130°, from phenylacetic acid.

Preparation 21

1-[4-(9-Bromononyloxy)-2-hydroxy-3-propylphenyl]ethanone

The *title compound* was prepared as an oil from 1-(2,4-dihydroxy-3-propylphenyl)ethanone and 1,9-di-bromononane by a similar procedure to Preparation 4.

Preparation 22

1-[4-(2-Heptenyloxy)-2-hydroxyphenyl]ethanone

The *title compound* was prepared as an oil from 1-(2,4-dihydroxyphenyl)ethanone and 1-bromo-2-heptene by a similar procedure to Preparation 12.

Preparation 23

1-[2,4-Dihydroxy-3-(1-methylhexyl)phenyl]ethanone

The compound of Preparation 22 (0.94 g) was heated at 190° for 3 h under nitrogen. The mixture was purified by chromatography on silica using a mixture of petroleum ether and ethyl acetate (3:1) as the eluent to give a solid (0.41 g). The crude alkene was hydrogenated in ethanol (30 ml) using 10% palladium oxide on charcoal as the catalyst. The mixture was filtered and the filtrate was evaporated to dryness to give a solid. The solid was recrystallised from a mixture of ethanol and water to give the *title compound* (0.22 g) with m.p. 78—81°.

Preparation 24

3-[3-Acetoxyphenyl]-2,3-dibromopropanoic acid, methyl ester

Bromine (3.7 ml) in carbon tetrachloride (150 ml) was added dropwise to a solution of 3-[3-acetoxyphenyl]-2-propenoic acid, methyl ester (15 g) in carbon tetrachloride (150 ml) illuminated with a 60 watt lamp. The addition was completed in 2.5 h and the solution was stirred for a further 2.5 h. The solution was evaporated to dryness and the residual solid was recrystallised from a mixture of methanol and water to give the *title compound* (19.5 g), with m.p. 84—86°.

Preparation 25

2-Bromo-3-(3-hydroxyphenyl)-2-propenoic acid

The compound of Preparation 24 (11.4 g) in dioxan (150 ml) was added to a solution of potassium hydroxide (15 g) in water (75 ml) and dioxan (75 ml). The solution was stirred for 20 h. The product mixture was acidified with 2 N hydrochloric acid, and extracted with ethyl acetate (2 x 500 ml). The combined organic extracts were evaporated to give an oil. The oil was crystallised from water to give the *title acid* (2.52 g) with m.p. 178—180°.

Preparation 26

2-Bromo-3-(3-hydroxyphenyl)-2-propenoic acid, methyl ester

Acetyl chloride (2 ml) was added cautiously to ice-cold methanol (40 ml). The compound of Preparation 25 (2 g) was added and the solution was heated under reflux for 5 h, cooled and

evaporated to dryness. The residual oil was dissolved in ethyl acetate and the solution was washed consecutively with 8% sodium bicarbonate solution and water, dried and evaporated. The residual solid was recrystallised from a mixture of methanol and water to give the *title ester* (1.39 g), with m.p. 97—99°.

Preparation 27

5-Bromo-2-hydroxy-4-[3-[[(4-methylphenyl)sulphonyl]oxy]propoxy]benzoic acid, methyl ester

A solution of bromine (0.1 ml) in chloroform (10 ml) was added to a solution of the compound of Preparation 17 (1 g) in chloroform (25 ml). The reaction mixture was stirred for 5 h and diluted with ether (200 ml). The solution was washed consecutively with 8% sodium bicarbonate solution, water and saturated brine dried and evaporated to dryness to give a white solid. The solid was recrystallised from a mixture of ethyl acetate and petroleum ether to give the *title compound* (1 g), with m.p. 141—142°.

Preparation 28

2-Hydroxy-4-[3-[[(4-methylphenyl)sulphonyl]oxy]propoxy]-5-nitrobenzoic acid, methyl ester

Nitric acid (1 ml) was added dropwise to a solution of the compound of Preparation 17 (1.5 g) in chloroform (20 ml). The reaction mixture was stirred for 16 h and then diluted with ethyl acetate (200 ml). The solution was washed consecutively with 8% sodium bicarbonate solution, water and saturated brine, dried and evaporated to give a red solid. The solid was recrystallised from a mixture of ethyl acetate and petroleum ether to give the *title compound* (0.55 g), with m.p. 143—145°.

Preparation 29

2-Hydroxy-4-[3-[(methylsulphonyl)oxy]propoxy]benzoic acid, methyl ester

Methanesulphonyl chloride (1.26 g) in dichloromethane (10 ml) was added dropwise over 15 min. to a solution of the compound of Preparation 16 (2.52 g) and triethylamine (1.5 g) in dichloromethane (30 ml) at 0 to −5°. The mixture was stirred for a further 15 min. at 0°, then washed successively with water, 2 N hydrochloric acid, 8% sodium bicarbonate solution and saturated brine, dried and evaporated. The residual solid was recrystallised from methanol to give the *title mesylate* (2.2 g), with m.p. 87—89°.

Preparation 30

2,4-Dihydroxybenzoic acid, phenylmethyl ester

2,4-Dihydroxybenzoic acid was dissolved in a solution of potassium carbonate (4.35 g) in water (50 ml) heated on a steam bath. The solution was evaporated to dryness and the residual solid washed well with acetone and dried to give the potassium salt (12.44 g). The potassium salt (12.44 g), benzylchloride (7.55 ml; 8.3 g) and triethylbenzylammonium chloride (0.93 g) were heated at 100° in dimethylformamide (120 ml) for 24 h. Water (120 ml) was added and the mixture was acidified and extracted with ether. The extracts were washed with water and 2% sodium bicarbonate solution, dried and evaporated. The residual oil was crystallised from cyclohexane to give the *title ester* (10.84 g), with m.p. 93—94°.

Preparation 31

2-Hydroxy-4-[3-hydroxypropoxy]benzoic acid, phenylmethyl ester

The compound of Preparation 30 (10.5 g), 3-bromo-1-propanol (3.9 ml; 5.99 g) and potassium carbonate (5.94 g) were heated under reflux in butanone (100 ml) for 24 h. Water (100 ml) and ethyl acetate (100 ml) were added and the organic phase was separated and washed consecutively with water, 1 N sodium carbonate and saturated brine and dried. The solution was evaporated to dryness and the residue (12.1 g) purified by chromatography on silica (600 g), using a mixture of petroleum ether and ethyl acetate (2:1) as the eluent. The fractions containing the major component were combined and evaporated to dryness to give the *title alcohol* as a white solid (5.7 g), with m.p. 65—66°.

Preparation 32

2-Hydroxy-4-[3-[[methylsulphonyl]oxy]propoxy]benzoic acid, phenylmethyl ester

A solution of methanesulphonyl chloride (0.56 ml; 0.83 g) in dichloromethane (10 ml) was added dropwise over 15 min to a solution of the compound of Preparation 31 (2 g) and triethylamine (1.38 ml; 1.0 g) in dichloromethane (30 ml) cooled in ice. The solution was stirred at room temperature for 15 min and washed with 1 N hydrochloric acid, 4% sodium bicarbonate and saturated brine, dried and evaporated to dryness. The residue or solid was recrystallised from methanol to give the *title mesylate* (2.23 g), with m.p. 84—85°.

Preparation 33

4-[3-[4-Acetyl-3-hydroxy-2-(2-propenyl)phenoxy]propoxy]-2-hydroxybenzoic acid, phenylmethyl ester

1-[2,4-Dihydroxy-3-(2-propenyl)phenyl]ethanone (1.01 g), the compound of Preparation 32

(2.0 g) and potassium carbonate (0.73 g) were heated under reflux in butanone (30 ml) for 24 h. The mixture was diluted with water (30 ml), the layers were separated and the aqueous phase was extracted with ethyl acetate. The organic phases were washed with saturated brine, dried and evaporated. The residual semi-solid was extracted with hot cyclohexane (200 ml) and the solid was evaporated to dryness. The residue was recrystallised three times from ethanol to give the *title ester* (1.28 g) with m.p. 111—112°.

Preparation 34

1-[2-Hydroxy-4-[3-[[methylsulphonyl]oxy]propoxy]-3-(2-propenyl)phenyl]ethanone

1-[2,4-Dihydroxy-3-(2-propenyl)phenyl]ethanone (4.65 g), 3-bromo-1-propanol (2.2 ml; 3.38 g) and potassium carbonate (3.34 g) were heated under reflux in butanone (50 ml) for 16 h. The mixture was cooled, diluted with water (50 ml) and acidified with 2 N hydrochloric acid. The layers were separated and the aqueous phase was extracted with ethyl acetate. The combined organic phases were washed with saturated brine, dried and evaporated to give the crude intermediate *alcohol* as an oil (5.8 g). A solution of methanesulphonyl chloride (1.7 ml; 2.52 g) in dichloromethane (30 ml) was added dropwise over 15 min to a solution of the crude alcohol (5.0 g) and triethylamine (4.2 ml; 3.05 g) in dichloromethane (90 ml) cooled in ice. The solution was stirred at room temperature for 30 min and washed with 1 N hydrochloric acid, 4% sodium bicarbonate and saturated brine, dried and evaporated. The residual oil was crystallised from methanol to give the *title mesylate* (3.03 g), with m.p. 81—82°.

Preparation 35

(a) 2-Methoxy-4-[3-[[(4-methylphenyl)sulphonyl]oxy]propoxy]benzoic acid, methyl ester

The compound of Preparation 17 (1 g) was added to 0.1 N sodium hydroxide solution (26.6 ml) and the mixture was evaporated to dryness. The residual solid was stirred at 25° in a mixture of dimethyl sulphate (8.75 ml), triethylbenzylammonium chloride (0.1 g) and butanone (50 ml) for 1.5 h. The mixture was acidified with 2 N hydrochloric acid and extracted with ether (3 × 25 ml). The combined organic extracts were washed with water and saturated brine, dried and evaporated to dryness. The residue was heated on a steam bath with 8% sodium bicarbonate solution (150 ml) and the cooled mixture was extracted with ethyl acetate. The organic extracts were dried and evaporated to give the *title compound* (0.51 g) as an oil, with λmax. 255 (ε 14,500).

Preparation 36

4-[3-[4-Acetyl-3-hydroxy-2-(2-propenyl)phenoxy]propoxy]-2-hydroxybenzoic acid, phenylmethyl ester

The compound of Preparation 34 (2.75 g), the compound of Preparation 30 (2.05 g) and potassium carbonate (1.16 g) were heated under reflux in butanone (70 ml) for 24 h. Water (70 ml) was added, the layers separated and the aqueous layer was extracted with ethyl acetate. The organic phases were washed with saturated brine, dried and evaporated. The residue was recrystallised from ethanol to give the *title ester* (3.22 g), with m.p. 111—112°.

Example 1

(a) 3-[3-(4-Acetyl-3-hydroxy-2-propylphenoxy)propoxy]benzoic acid

Sodium hydride (oil free; 0.18 g) was added to a soution of 3-hydroxybenzoic acid, methyl ester (1.12 g) dissolved in dry dimethylformamide (25 ml). The mixture was stirred at 25° for 15 min. and sodium iodide (0.5 g) and 1-[4-(3-chloropropoxy)-2-hydroxy-3-propylphenyl]ethanone (1.0 g) added. The mixture was heated at 110° for 3 hours cooled and evaporated to dryness. The residual oil was triturated with water and purified by chromatography on silica using a mixture of ethyl acetate and petroleum ether (1:2) as the eluent to give the methyl ester of the *title compound* (0.7 g).

The ester was dissolved in N/3 ethanolic sodium hydroxide (30 ml) and the solution heated under reflux for 20 minutes. The solvent was removed under reduced pressure and the residue washed with ethyl acetate and dissolved in water. The solution was acidified with 2 N hydrochloric acid and the solid filtered off and recrystallised from a mixture of ethanol and water to give the *title acid* (0.5 g) with m.p. 138—139°.

The following compounds were prepared by a similar procedure from 1-[4-(3-chloropropoxy)-2-hydroxy-3-propylphenyl]ethanone and the appropriate ester:—

(b) 3-[4-[3-(4-Acetyl-3-hydroxy-2-propylphenoxy)propoxy]phenyl]-2-propenoic acid, m.p. 176—179° (from aqueous acetone) (methyl ester had m.p. 135—137° (from aqueous ethanol)); from 3-(4-hydroxyphenyl)-2-propenoic acid, methyl ester.

(c) 3-[3-(4-Acetyl-3-hydroxy-2-propylphenoxy)propoxy]benzeneacetic acid, m.p. 81.5—82° (from petroleum ether); from 3-hydroxybenzeneacetic acid, ethyl ester.

(d) 4-[3-(4-Acetyl-3-hydroxy-2-propylphenoxy)propoxy]benzoic acid, m.p. 186—187.5° (from aqueous acetone); from 4-hydroxybenzoic acid, methyl ester.

(e) 3-[3-(4-Acetyl-3-hydroxy-2-propylphenoxy)propoxy]benzenepropanoic acid, m.p. 71—72° (from a mixture of dilute hydrochloric acid and methanol); from 3-hydroxy-benzenepropanoic acid, methyl ester.

(f) 2-[3-[3-(4-Acetyl-3-hydroxy-2-propylphenyl]propoxy]phenoxy]acetic acid, m.p. 117—118° (from

aqueous ethanol); from 3-hydroxyphenoxyacetic acid, methyl ester.

(g) 2-[4-[3-(4-Acetyl-3-hydroxy-2-propylphenyl)propoxy]phenoxy]acetic acid, m.p. 123—124° (from aqueous ethanol); from 4-hydroxyphenoxyacetic acid, ethyl ester.

Example 2

(a) 3-[3-[3-(4-Acetyl-3-hydroxy-2-propylphenoxy)propoxy]phenyl]-2-propenoic acid

A mixture of 3-(3-hydroxyphenyl)-2-propenoic acid, methyl ester (4.9 g), the compound of Preparation 5 (10 g) and potassium carbonate (4 g) in butanone (250 ml) was heated under reflux for 24 hours. The mixture was reduced to dryness and the residue extracted with ethyl acetate. The extracts were evaporated to give an oil which was crystallised from a mixture of ethanol and water to give the methyl ester of the *title compound* (8.9 g) with m.p. 88—89°.

A mixture of the ester (7.8 g) lithium hydroxide monohydrate (4 g), tetrahydrofuran (30 ml) and water (30 ml) was stirred at 25° for 5 hours and diluted with water (200 ml). The aqueous solution was washed with ether (3 × 75 ml) and acidified with 2 N hydrochloric acid. The precipitate was filtered off and recrystallised from ethanol to give the *title acid* (5.13 g) with m.p. 151—153°.

The following compounds were prepared by a similar procedure:

(b) 5-[3-(4-Acetyl-3-hydroxy-2-propylphenoxy)propoxy]benzene-1,3-dicarboxylic acid, m.p. 224—225° (from aqueous ethanol); from 5-hydroxy-1,3-benzenedicarboxylic acid, dimethyl ester and the compound of Preparation 5.

(c) 4-[3-(4-Acetyl-3-hydroxy-2-propylphenoxy)propoxy]-2-hydroxy-3-propylbenzoic acid, m.p. 170—172° from aqueous ethanol); from the compound of Preparation 5 and the compound of Preparation 3.

(d) 3-[3-[4-(4-Acetyl-3-hydroxy-2-propylphenoxy)methylphenyl]methoxyphenyl]-2-propenoic acid, m.p. 163—164° (from ethanol) (methyl ester had m.p. 110—111° (from a mixture of ethyl acetate and petroleum ether)); from 3-(3-hydroxyphenyl-2-propenoic acid, methyl ester and the compound of Preparation 4a.

(e) 3-[3-[4-(4-Acetyl-3-hydroxy-2-propylphenoxy)butoxy]phenyl]-2-propenoic acid, m.p. 115—117° (from a mixture of ethyl acetate and petroleum ether; from the compound of Preparation 7a and 3-(3-hydroxyphenyl)-2-propenoic acid, methyl ester.

(f) 3-[3-[2-(4-Acetyl-3-hydroxy-2-propylphenoxy)ethoxy]phenyl]-2-propenoic acid, m.p. 120—121° (from a mixture of ethyl acetate and petroleum ether); from the compound of Preparation 7b and 3-(3-hydroxyphenyl)-2-propenoic acid, methyl ester.

(g) 3-[3-[4-(4-Acetyl-3-hydroxy-2-propylphenoxy)-2-butynyloxy]phenyl]-2-propenoic acid, m.p. 146—147° (from a mixture of ethyl acetate and petroleum ether) (methyl ester had m.p. 83—84° (from a mixture of petroleum ether and ethyl acetate)); 3-(3-hydroxyphenyl)-2-propenoic acid, methyl ester and the compound of Preparation 4b.

(h) 3-[2-[3-(4-Acetyl-3-hydroxy-2-propylphenoxy)propoxy]phenyl]-2-propenoic acid, m.p.145—147° (from aqueous ethanol) (methyl ester had m.p. 78—80°) from aqueous ethanol); from the compound of Preparation 5 and 3-(2-hydroxyphenol)-2-propenoic acid, methyl ester.

(i) 3-[5-[3-(4-Acetyl-3-hydroxy-2-propylphenoxy)propoxy]-2-bromophenyl]-2-propenoic acid, m.p. 168—169° (from aqueous methanol); from the compound of Preparation 5 and the compound of Preparation 9.

Example 3

3-[3-[3-(4-Acetyl-3-hydroxy-2-propylphenoxy)propoxy]phenyl]-N-1H-tetrazole-5-yl-2-propenamide

The compound of Example 2 (0.5 g) and N,N'-carbonyldiimidazole (0.45 g) were stirred at 25° in a mixture of dry tetrahydrofuran (15 ml) and dry dimethylformamide (1 ml) for 17 hours. A solution of 5-aminotetrazole (0.4 g) in dimethylformamide (10 ml) was added and the mixture stirred at 25° for 4 hours and at 90° for 2½ hours. The mixture was cooled and diluted with water (100 ml) and the precipitate filtered off, washed with ether and recrystallised from a mixture of dimethylformamide and water to give the *title amide* (0.29 g) with m.p. 234—236°.

Example 4

(a) 3-[3-[3-(4-Acetyl-3-hydroxy-2-(2-propenyl)phenoxy]propoxy]phenyl]-2-propenoic acid

1-[2,4-Dihydroxy-3-(2-propenyl)phenyl]ethanone (1.5 g), the compound of Preparation 6a (2.7 g) and potassium carbonate (2.5 g) were heated under reflux in butanone (25 ml) for 8 hours. The mixture was evaporated to dryness and the residue dissolved in a mixture of 2 N hydrochloric acid and ethyl acetate. The organic layer was washed with 2 N sodium carbonate solution, dried and evaporated to dryness. The residue was recrystallised from ethanol to give the methyl ester of the *title compound* (2.6 g) with m.p. 89—90°.

A mixture of the ester (1.5 g), lithium hydroxide monohydrate (0.8 g), water (15 ml) and tetrahydrofuran (15 ml) was stirred at room temperature for 20 hours. The mixture was washed with ether (2 × 10 ml) and the aqueous layer acidified. The precipitate was filtered off and recrystallised from ethanol to give the *title acid* (1.2 g) with m.p. 127.5—128.5°.

(b) 3-[3-[5-(4-Acetyl-3-hydroxy-2-propylphenoxy)pentyloxy]phenyl]-2-propenoic acid, m.p. 126—127° (from

aqueous ethanol); from 1-[2,4-dihydroxy-3-propylphenyl]ethanone and the compound of Preparation 6b.

(c) 3-[3-[3-(4-Acetyl-3-hydroxy-2-methylphenoxy]propoxy]phenyl]-2-propenoic acid, m.p. 157.5—159° (from ethanol) (methyl ester had m.p. 87—89° (from ethanol)); from 1-(2,4-dihydroxy-3-methylphenyl)-ethanone and the compound of Preparation 6a.

(d) 3-[3-[3-(4-Acetyl-3-hydroxy-2,6-dipropylphenoxy)propoxy]phenyl]-2-propenoic acid, m.p. 123.5—125° (from aqueous ethanol); from the compound of Preparation 6a and the compound of Preparation 8.

(e) 3-[3-[3-[4-Acetyl-3-hydroxy-2-(1-methylpropyl)phenoxy]propoxy]phenyl]-2-propenoic acid, m.p. 126.5—129°, (from aqueous ethanol); from the compound of Preparation 14 and the compound of Preparation 6a.

## Example 5

3-[3-(4-Acetyl-3-hydroxy-2-propylphenoxy)propoxy]benzene propanoic acid

The compound of Example 2 (0.37 g) was hydrogenated in methanol (25 ml) using 10% palladium oxide on charcoal as catalyst. The mixture was filtered and the filtrate evaporated to dryness to give a brown oil which was purified by chromatography on silica (15 g) using a mixture of petroleum ether and ethyl acetate (2:1) as the eluent. The product was crystallised from a mixture of methanol and 10% aqueous acetic acid to give the *title acid* (0.09 g) with m.p. 72—73°.

## Example 6

3-[3-(4-Acetyl-3-hydroxy-2-propylphenoxymethoxy)phenyl]-2-propenoic acid

A mixture of 1-(2,4-dihydroxy-3-propylphenyl)ethanone (7 g), 3-(3-hydroxyphenyl)-2-propenoic acid, methyl ester (6.4 g), dibromomethane (30 g) and potassium carbonate (60 g) was heated under reflux in butanone (200 ml) for 18 hours. The cooled mixture was filtered, diluted with ether (200 ml) and washed with water and 2 N sodium hydroxide solution. The solution was dried and evaporated to give an oil which was purified by chromatography on silica using dichloromethane as the eluent to give the methyl ester of the *title compound* (3.2 g) which had m.p. 87—87.5° after recrystallisation from petroleum ether.

A mixture of the ester (1.5 g), lithium hydroxide monohydrate (1 g), tetrahydrofuran (30 ml) and water (30 ml) was stirred at 25° for 4 hours. The reaction mixture was washed with ether, acidified with 2 N hydrochloric acid and extracted with ethyl acetate. The extracts were dried and evaporated and the residue recrystallised from a mixture of petroleum ether and ethyl acetate to give the *title acid* with m.p. 174—175°C.

## Example 17

3-[3-[4-(4-Acetyl-3-hydroxy-2-propylphenoxy)-2-butenyloxy)phenyl]-2-propenoic acid

The compound of Example 2g (0.95 g) was hydrogenated in pyridine (20 ml) using 5% palladium on barium sulphate as catalyst. The catalyst was filtered off after the theoretical volume of hydrogen had been absorbed and the filtrate evaporated to dryness. The residue was recrystallised from a mixture of ethyl acetate and petroleum ether (b.p. 60—80°) to give the *title acid* (0.72 g) with m.p. 126—129°.

## Example 8

4-[3-(4-Acetyl-3-hydroxy-2-propylphenoxy)propoxy]-2-hydroxybenzoic acid.

Methyl 2,4-dihydroxybenzoate (5 g), the compound of Preparation 5 (10.8 g) and potassium carbonate (4.5 g) were heated under reflux in butanone (700 ml) for 26 h. The mixture was filtered and the filtrate evaporated to dryness. The residue was recrystallised from methanol to give the methyl ester of the *title compound* (8.6 g) with m.p. 95—96°.

The ester (1 g) and sodium hydroxide (0.48 g) were heated under reflux in a mixture of methanol (63 ml) and water (5 ml) for 14 h. The solution was evaporated to dryness and the residue dissolved in water. The solution was acidified and extracted with ethyl acetate. The extracts were dried and evaporated and the residue recrystallised from a mixture of acetone and water to give the *title compound* (0.8 g) with m.p. 173—174°.

## Example 9

(a) 3-[5-[3-[4-Acetyl-3-hydroxy-2-propylphenoxy]propoxy]-2-nitrophenyl]-2-propenoic acid.

3-[5-Hydroxy-2-nitrophenyl]-2-propenoic acid, methyl ester (3 g), the compound of Preparation 11 (5.5 g) and potassium carbonate (3.75 g) were heated under reflux in butanone (150 ml) for 6 h. The mixture was cooled, acidified with 2 N hydrochloric acid and extracted with ethyl acetate. The extracts were washed with 2 N sodium carbonate (2 × 200 ml) and water (2 × 200 ml), dried and evaporated to give the crude methyl ester of the *title compound* as an oil (6 g).

The crude ester (4.6 g) and lithium hydroxide monohydrate (2 g) were stirred in a mixture of water (40 ml) and tetrahydrofuran (40 ml) for 6 h. The mixture was diluted with water (200 ml), washed with ether and the aqueous layer acidified and extracted with ethyl acetate. The extracts were dried and

evaporated to dryness and the residue recrystallised from a mixture of ethyl acetate and petroleum ether to give the *title compound* (3.2 g) with m.p. 158—160°.

The following compounds were prepared by a similar procedure:

(b) 3-[3-[3-[4-Acetyl-3-hydroxy-2-propylphenoxy]propoxy]-4-nitrophenyl]-2-propenoic acid, m.p. 191—194° (from ethanol); from 3-[3-hydroxy-4-nitrophenyl]-2-propenoic acid, methyl ester and the compound of Preparation 11.

(c) 3-[3-[3-[4-Acetyl-3-hydroxy-2-propylphenoxy]propoxy]-4-acetylphenyl]-2-propenoic acid, m.p. 200—202° (from aqueous ethanol); from the compound of Preparation 15 and the compound of Preparation 11.

(d) 5-[3-[4-Acetyl-3-hydroxy-2-propylphenoxy]propoxy]-3-hydroxybenzoic acid, m.p. 170—172°; from methyl 3,5-dihydroxybenzoate and the compound of Preparation 11.

Example 10

(a) 5-[2-[3-[3-[4-Acetyl-3-hydroxy-2-propylphenoxy]propoxy]ethenyl]-1H-tetrazole hemihydrate

3-[3-Hydroxyphenyl]propenonitrile (2 g), the compound of Preparation 11 (5.56 g) and potassium carbonate (3.78 g) were heated under reflux in butanone (70 ml) for 8 h. The mixture was acidified with hydrochloric acid and extracted with ethyl acetate. The extracts were dried and evaporated and the residue recrystallised from cyclohexane to give the intermediate nitrile (3.7 g) with m.p. 110—111°.

The nitrile (1 g), ammonium chloride (0.14 g) and sodium azide (0.18 g) were heated in dimethylformamide (25 ml) at 120° for 21 h. The mixture was cooled, diluted with water (200 ml) and acidified with 2 N hydrochloric acid. The mixture was extracted with ethyl acetate and the extracts dried and evaporated to dryness. The residue was heated at 100° with 5 N hydrochloric acid for 0.75 h. The mixture was cooled and the solid filtered off and recrystallised from acetic acid to give the *title compound* (0.48 g) with m.p. 102—104°.

The following compound was prepared by a similar procedure.

(b) 5-[3-[3-[4-Acetyl-3-hydroxy-2-propylphenoxy]propoxy]phenyl]-1H-tetrazole, m.p. 154—156° (from a mixture of water and ethanol); from 3-cyanophenol and the compound of Preparation 11 (the intermediate nitrile had m.p. 60—61° (from cyclohexane)).

Example 11

a) 4-[3-(4-Acetyl-3-hydroxy-2-propylphenoxy)propoxy]-2-hydroxybenzoic acid

Methyl 2,4-dihydroxybenzoate (7.03 g), the compound of Preparation 11 (17 g) and potassium carbonate (11.5 g) were heated under reflux in butanone (300 ml) for 20 h. The mixture was cooled, diluted with water (150 ml) and the layers separated. The aqueous phase was extracted with ethyl acetate and the combined organic phases dried and evaporated to dryness. The residue was recrystallised from methanol to give the methyl ester of the *title compound* (13.56 g) with m.p. 95—96°.

The ester (13.5 g) and sodium hydroxide (4.5 g) were heated under reflux in a mixture of methanol (780 ml) and water (65 ml) for 21 h. The mixture was acidified, the methanol distilled off under reduced pressure and the resulting suspension filtered. The solid was washed with water, dissolved in hot acetone (130 ml) and the solution diluted with water (80 ml). The crystals which formed on cooling were filtered off and recrystallised from toluene to give the *title compound* (9.9 g) with m.p. 176—177°.

The following compounds were prepared by a similar procedure from the compound of Preparation 11 and the appropriate ester:

b) 4-(3-[4-Acetyl-3-hydroxy-2-propylphenoxy]propoxy]-2-hydroxy-3-(2-propenyl)benzoic acid, m.p. 178—180° (from a mixture of ethanol and water) (methyl ester had m.p. 84—86° (from a mixture of ethanol and water)); from the compound of Preparation 2.

c) 4-[3-(4-Acetyl-3-hydroxy-2-propylphenoxy)propoxy]-2-hydroxy-3-methyl-benzoic acid hemihydrate, m.p. 177—179° (from a mixture of ethanol and water) (ethyl ester had m.p. 110—113°); from 2,4-dihydroxy-3-methylbenzoic acid, ethyl ester.

d) 3-[3-(4-Acetyl-3-hydroxy-2-propylphenoxy)propoxy]-2-hydroxybenzoic acid, m.p. 172° (from a mixture of ethanol and water) (methyl ester had m.p. 93—96° (from a mixture of ethyl acetate and petroleum ether)): from 2,3-dihydroxybenzoic acid, methyl ester.

e) 5-[3-(4-Acetyl-3-hydroxy-2-propylphenoxy)propoxy]-2-hydroxybenzoic acid, m.p. 155—156° (from a mixture of ethanol and water); from 2,5-dihydroxybenzoic acid, ethyl ester.

Example 12

a) 4-[3-(4-Acetyl-3-hydroxy-2-propylphenoxy)propoxy]-2-hydroxybenzoic acid

1-(2,4-Dihydroxy-3-propylphenyl)ethanone (3 g), the compound of Preparation 17 (3.8 g) and potassium carbonate (1.38 g) were heated under reflux in butanone (300 ml) for 16 h. The mixture was acidified with 2 N hydrochloric acid and extracted with ethyl acetate. The extracts were washed consecutively with water, 8% sodium bicarbonate solution and saturated brine, dried and evaporated to give the crude methyl ester of the *title compound* (3.6 g) as an oil.

The ester (3.6 g) was heated under reflux in a mixture of ethanol (30 ml) and 2 N sodium

hydroxide (200 ml) for 5 h. The mixture was acidified and extracted with ethyl acetate. The extracts were washed with water and saturated brine, dried and evaporated. The residue was recrystallised once from a mixture of ethanol and water and once from toluene to give the *title compound* (1.8 g), with m.p. 175—176°.

The following compounds were prepared by a similar procedure:

b) 4-[3-[4-Acetyl-3-hydroxy-2-[(1-methylhexyl)phenoxy]propoxy]-2-hydroxybenzoic acid, m.p. 106—108° (from a mixture of ethanol and water); from the compound of Preparation 17 and the compound of Preparation 23.

c) 4-[3-(3-Hydroxy-4-(1-oxopropyl)-2-propylphenoxy]propoxyl]-2-hydroxybenzoic acid, m.p. 155—158° (from a mixture of ethanol and water) (methyl ester had m.p. 79—80° (from a mixture of ethanol and water)); from the compound of Preparation 17 and the compound of Preparation 20b.

d) 4-[3-[3-Hydroxy-4-phenylacetyl-2-propylphenoxy]propoxy]-2-hydroxybenzoic acid, m.p. 167—169° (methyl ester had m.p. 102—103° (from ethanol)); from the compound of Preparation 17 and the compound of Preparation 20c.

e) 4-[3-[4-Benzoyl-3-hydroxy-2-propylphenoxy]propoxy]-2-hydroxybenzoic acid, m.p. 91—92° (from toluene) (methyl ester had m.p. 117—118° (from ethanol)); from the compound of Preparation 17 and the compound of Preparation 20a.

Example 13

3-[3-[3-(4-Acetyl-3-hydroxy-2-propylphenoxy)propoxy]phenyl]-2-propynoic acid

The compound of Preparation 11 (1.62 g) the compound of Preparation 26 (1.03 g) and potassium carbonate (1.21 g) were heated under reflux in butanone (120 ml) for 24 h. The mixture was cooled, filtered and the filtrate was evaporated to dryness. The resulting oil was purified by chromatography on silica using a mixture of petroleum ether and ethyl acetate (3:1) as the eluent to give the methyl ester of the *title compound* (1.1 g), with m.p. 56—58°.

A mixture of the ester (0.91 g), lithium hydroxide monohydrate (0.373 g), tetrahydrofuran (20 ml) and water (20 ml) was stirred at room temperature from 6 h, washed with ether (2 x 50 ml) and acidified with 2 N hydrochloric acid. The mixture was extracted with ethyl acetate (2 x 30 ml). The extracts were dried and evaporated to give a solid which was recrystallised twice from a mixture of ethyl acetate and petroleum ether to give the *title acid* (0.372 g), with m.p. 153—156°.

Example 14

4-[3-(4-Acetyl-3-hydroxy-2-propylphenoxy)-2-hydroxypropoxy]-2-hydroxybenzoic acid

A mixture of 1-[2-hydroxy-4-(oxiranylmethoxy)-3-propylphenyl]ethanone (1 g), 2,4-dihydroxybenzoic acid, methyl ester (0.672 g) and benzyltrimethylammonium hydroxide (1 drop) in dimethylformamide (40 ml) was heated under reflux for 6 h. The mixture was reduced to dryness and the residue was dissolved in ethyl acetate. The solution was washed consecutively with water, 1 N sodium hydroxide solution, water and saturated brine, dried and evaporated to give an oil. The oil was purified by chromatography on silica using a mixture of petroleum ether and ethyl acetate (2:1) as the eluent. The fractions containing the component which had an Rf of 0.33 on t.l.c. (silica; petroleum ether:ethyl acetate 2:1) were collected and evaporated to dryness to give the methyl ester of the *title compound* (0.52 g), with m.p. 126—128°.

The ester (0.418 g), sodium hydroxide (0.2 g), methanol (30 ml) and water (2 ml) were heated under reflux for 24 h. The methanol was removed under reduced pressure, the residual aqueous solution was acidified with 2 N hydrochloric acid and extracted with ethyl acetate (2 x 25 ml). The extracts were washed with water and saturated brine, dried and evaporated. The residual solid was recrystallised from a mixture of water and ethanol to give the *title acid* (0.342 g), with m.p. 177—180°.

Example 15

4-[3-(4-Acetyl-3-hydroxy-2-propylphenoxy)propoxy]-2-hydroxy-N-1H-tetrazol-5-yl-benzamide

The *title compound* (m.p. 248—250°, from a mixture of water and dimethylformamide) was prepared by a similar procedure to Example 3, from the compound of Example 12a.

Example 16

4-[9-(4-Acetyl-3-hydroxy-2-propylphenoxy)nonyloxy]-2-hydroxybenzoic acid hemihydrate

The *title compound*, m.p. 92—94° (from a mixture of ethanol and 0.1 N hydrochloric acid) (methyl ester had m.p. 70—72°); was prepared by a similar procedure to Example 2 from the compound of Preparation 21 and 2,4-dihydroxybenzoic acid, methyl ester.

Example 17

4-[3-[4-Acetyl-3-hydroxy-2-propylphenoxy]propoxy]-2-hydroxybenzoic acid

The compound of Preparation 36 (1.1 g) was hydrogenated at atmospheric pressure in ethanol (75 ml) using 5% platinum on charcoal (45 mg) as the catalyst. When the uptake of hydrogen was complete the mixture was filtered and the filtrate was evaporated to dryness. The residue was

recrystallised from toluene to give the *title acid* (0.78 g), with m.p. 173—174°.

Example 18

4-[3-[4-Acetyl-3-hydroxy-2-(2-propenyl)phenoxy]propoxy]-2-hydroxybenzoic acid

The compound of Preparation 36 (1.0 g) was heated under reflux with 2 N sodium hydroxide (3 ml) and methanol (30 ml) for 24 h. The mixture was poured into water (100 ml), acidified and the solid was filtered off, dried and recrystallised from toluene to give the *title acid* (0.62 g), with m.p. 180—181° (dec.).

Example 19

4-[3-(4-Acetyl-3-hydroxy-2-propylphenoxy)propoxy]-2-hydroxybenzoic acid

1-(2,4-Dihydroxy-3-propylphenyl)ethanone (0.51 g), the compound of Preparation 29 (1 g) and potassium carbonate (0.83 g) were heated under reflux in butanone (20 ml) for 7.25 h. The mixture was cooled and filtered. The residue was washed with butanone and the combined filtrate and washings were evaporated to give a solid which was recrystallised from methanol to give the methyl ester of the *title compound* (0.8 g), with m.p. 99—100.5°.

The ester (0.76 g) was heated under reflux with 2 N sodium hydroxide (4.75 ml) and methanol (50 ml) for 18 h. The mixture was evaporated to dryness and the residue dissolved in water (100 ml). The solution was washed with ethyl acetate, acidified with 2N hydrochloric acid and extracted with ethyl acetate (3 x 50 ml). The combined extracts were washed with water and saturated brine, dried and evaporated to dryness. The solid residue was recrystallised three times from toluene, with charcoal decolourisation, to give the *title acid* (0.4 g), with m.p. 173—175°.

Example 20

4-[3-(4-Acetyl-3-hydroxy-2-propylphenoxy)propoxy]-2-methoxybenzoic acid

The *title compound*, m.p. 110—112° (from a mixture of petroleum ether and toluene) (methyl ester had m.p. 79—81°); was prepared by a similar procedure to Example 12a from the compound of Preparation 35 and 1-[2,4-dihydroxy-3-propylphenyl]ethanone.

Example 21

4-[3-(4-Acetyl-3-hydroxy-2-propylphenoxy)propoxy]-5-bromo-2-hydroxybenzoic acid

The *title compound* m.p. 172—173° (from toluene) (methyl ester had m.p. 120—122° (from a mixture of petroleum ether and ethyl acetate)); was prepared by a similar procedure to Example 12a from the compound of Preparation 27 and 1-[2,4-dihydroxy-3-propylphenyl]ethanone.

Example 22

4-[3-(4-Acetyl-3-hydroxy-2-propylphenoxy)propoxy]-2-hydroxy-5-nitrobenzoic acid

The *title compound* m.p. 197—198° (from a mixture of ethanol and water) (methyl ester had m.p. 89—91° (from a mixture of petroleum ether and ethyl acetate)); was prepared by a similar procedure to Example 12a from the compound of Preparation 28 and 1-[2,4-dihydroxy-3-propylphenyl]ethanone.

Pharmaceutical Examples

| Tablets | Direct Compression | mg/tablet |
|---|---|---|
| | Active ingredient | 100.00 |
| | Microcrystalline Cellulose B.P.C. | 298.00 |
| | Magnesium Stearate | 2.00 |
| | Compression Weight | 400.00 |

The active ingredient is sieved through a 250 μm sieve, blended with the excipients and compressed using 10.0 mm punches.

| Capsules | | mg/capsule |
|---|---|---|
| | Active Ingredient | 100.00 |
| | STA-RX 1500 | 99.00 |
| | Magnesium Stearate B.P. | 1.00 |

The active ingredient is sieved through a 250 $\mu$m sieve and blended with the other materials. The mix is filled into No. 2 hard gelatin capsules.

| Inhalation Cartridges | | cartridge |
|---|---|---|
| | Active Ingredient (micronised | 10 mg |
| | Lactose B.P. to | 25 mg |

The active ingredient is micronised so that the majority of particles are between 1 $\mu$m and 5 $\mu$m in longest dimension and none are greater then 10 $\mu$m. The active ingredient is then blended with the lactose and the mix is filled into No. 3 hard gelatin capsules.

The ability of the compounds to act as antagonists of the action of SRS-A was shown using guinea-pig isolated ileum according to the methods of Coleman *et al*, Br. J. Pharmacol., (1979), *66*, 83P. Compounds were tested at concentrations of $10^{-6}$ M and $pA_2$ values were determined using the standard procedures of Arunlakshana and Schild, Br. J. Pharmac. Chemother., (1959), *14*, 48—58:

| Example No. | $pA_2$ | Example No. | $pA_2$ |
|---|---|---|---|
| 1g | 7.0 | 5 | 7.3 |
| 2a | 7.4 | 8 | 7.8 |
| 2c | 7.4 | 9b | 7.5 |
| 4b | 7.3 | 10b | 7.1 |

The compounds are in general non-toxic at therapeutically useful doses. For example, the compounds of Examples 2a, 5 and 8, when given orally to dogs at doses of up to 10 mg/kg, produced no adverse effects.

**Claims for the Contracting States: BE CH DE FR GB IT LI NL SE**

1. A compound of the general formula (I)

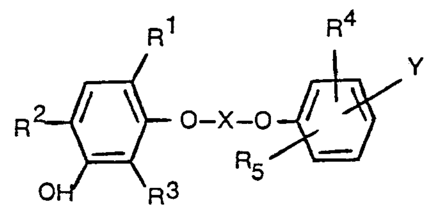

(I)

wherein

Y represents the group —A, —ZA or $OZ^1A$ wherein A represents a carboxylic acid group, a 5-1H-tetrazolyl ring, or N-5-1H-tetrazolylcarboxamide group;

Z represents a $C_{1-4}$ alkylene, $C_{2-4}$ alkenylene or $C_{2-4}$ alkynylene chain optionally substituted by one or more $C_{1-3}$ alkyl groups and $Z^1$ represents a $C_{1-4}$ alkylene chain optionally substituted by one or more $C_{1-3}$ alkyl groups:

X represents a $C_{1-10}$ carbon chain which may be saturated or unsaturated in the case of chains containing at least 4 carbon atoms, and may be substituted by a hydroxy group or by one or more $C_{1-3}$ alkyl groups, which chain may be interrupted by a benzene ring which may be linked through its 1- and 2-, 1- and 3-, or 1- and 4-positions;

$R^1$ represents a hydrogen atom or a $C_{1-6}$ alkyl group;

$R^2$ represents the group —$COR^6$ where $R^6$ represents a hydrogen atom, an aryl group, or a $C_{1-6}$ alkyl group which may be substituted by an aryl group;

$R^3$ represents a $C_{1-6}$ alkyl group or a $C_{3-6}$ alkenyl group; and

$R^4$ and $R^5$, which may be the same or different, each represents a hydrogen atom, a halogen atom or a hydroxy, $C_{1-3}$ alkoxy, $C_{1-6}$ alkyl, $C_{3-6}$ alkenyl, $C_{1-3}$ alkanoyl, nitro or carboxylic acid group, with the proviso that $R^4$ and $R^5$ cannot both be nitro, alkanoyl or carboxylic acid groups,
and physiologically acceptable salts thereof.

2. A compound according to claim 1, wherein in the general formula (I): Y represents a carboxylic acid group or a 5-1H-tetrazolyl ring or the group ZA or $OZ^1A$ where A is a carboxylic acid group and Z is a $C_{1-4}$ alkylene chain, a $C_{2-4}$ alkenylene chain or a $C_{2-4}$ alkynylene chain and $Z^1$ is methylene.

3. A compound according to claim 1 or 2, wherein in the general formula (I): X represents a straight carbon chain of formula —$(CH_2)_n$ where *n* is a number from 1 to 10, or a group of formula —$CH_2X^1CH_2$— where X is a benzene ring linked through its 1- and 2-, 1- and 3-, or 1- and 4-positions.

4. A compound according to any of claims 1 to 3, wherein in the general formula (I): $R^1$ represents a hydrogen atom or a $C_{3-6}$ alkyl group.

5. A compound according to any of claims 1 to 4, wherein in the general formula (I): $R^2$ represents the group $COR^6$ where $R^6$ represents a $C_{1-6}$ alkyl group.

6. A compound according to any of claims 1 to 5, wherein in the general formula (I): $R^3$ represents a $C_{3-6}$ alkyl or $C_{3-6}$ alkenyl group.

7. A compound according to any of claims 1 to 6, wherein in the general formula (I): $R^4$ represents a hydrogen or halogen atom or a hydroxy, nitro or $C_{1-3}$ alkanoyl group.

8. A compound according to any of claims 1 to 7, wherein in the general formula (I): $R^5$ represents a hydrogen atom or a $C_{1-6}$ alkyl group or a $C_{2-4}$ alkenyl group.

9. A compound according to any of claims 1 to 8, wherein in the general formula (I), $R^4$ represents a hydrogen atom or a hydroxyl group and $R^5$ is a hydrogen atom.

10. A compound of the general formula (II)

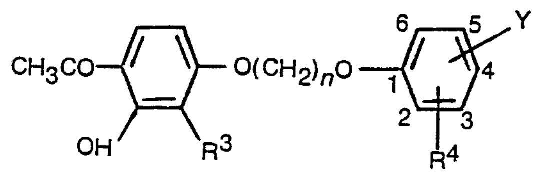
(II)

wherein Y is a carboxylic acid group or a 5-1H-tetrazolyl ring or a N-5-1H-tetrazolylcarboxamide group, or a $C_{1-2}$ alkyl, $C_{1-2}$ alkoxy or $C_2$-alkenyl chain terminally substituted by a carboxylic acid group;

$R^3$ represents $C_{3-6}$ alkyl or $C_{3-6}$ alkenyl group,

$R^4$ represents a hydrogen or bromine atom, or a hydroxy, nitro or acetyl group, and *n* is 1, 3 or 5, and physiologically acceptable salts thereof.

11. A compound according to claim 10, wherein in the general formula (II) Y, is situated at the 3- or 4-position in the benzene ring.

12. A compound according to claim 10 or 11, wherein in the general formula (II) *n* is 3.

13. 4-[3-(4-Acetyl-3-hydroxy-2-propylphenoxy)propoxy]-2-hydroxybenzoic acid and its physiologically acceptable salts.

14. 4-[3-(4-Acetyl-3-hydroxy-2-propylphenoxy)propoxy]-2-hydroxy-N-1H-tetrazol-5-y-benzamide and its physiologically acceptable salts.

15. 3-[3-[3-(4-Acetyl-3-hydroxy-2-methylphenoxy)propoxy]phenyl]-2-propenoic acid and its physiologically acceptable salts.

16. A pharmaceutical composition comprising, as active ingredient, one or more compounds according to any of claims 1 to 15 together with one more physiologically acceptable carriers or excipients therefor.

17. A pharmaceutical composition according to claim 16 which is formulated as a powder in a form suitable for administration by inhalation.

18. A process for the preparation of a compound of general formula (I) as defined in claim 1 or a physiologically acceptable salt thereof which comprises the steps:

A(i) in order to prepare a compound of general formula (1), in which —Y represents the group —$CH{=}CHCO_2H$, subjecting a compound of general formula (III)

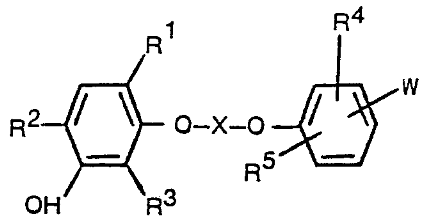
(III)

in which $R^1$ to $R^5$ and X are as defined in claim 1 and W represents —CHO, to the Perkin synthesis of cinnamic acids; or

A(ii) in order to prepare a compound of general formula (I) in which Y represents the group —A, —ZA or —$OZ^1A$ where Z and $Z^1$ are as defined in claim 1 and A is a carboxylic acid group, subjecting a compound of general formula (III) where W represents the group —E, —ZE or —$OZ^1E$ where E is a carboxylic ester, amide or nitrile group, to acid or base hydrolysis; or

A(iii) in order to prepare a compound of general formula (I) where Y represents the group —A, —ZA or $OZ^1A$ where Z and $Z^1$ are as defined in claim 1 and A is a 5-1H-tetrazolyl ring, reacting a compound of general formula (III) in which W represents —E, —ZE or $OZ^1E$ and E represents a nitrile, with sodium azide and ammonium chloride; or

A(iv) in order to prepare a compound of general formula (I) in which Y represents the group —A, —ZA or —$OZ^1A$ where A is a carboxylic acid group and Z and $Z^1$ are as defined in claim 1, subjecting to hydrogenolysis a compound of general formula (III) in which W represents the group —E, —ZE or —$OZ^1E$ and E is a carboxylic ester group of formula —$COOR^7$ where $R^7$ is a group removable by hydrogenolysis; or

A(v) in order to prepare a compound of general formula (I) in which Y represents the group —ZA where Z is a $C_{2-4}$ alkylene or $C_{2-4}$ alkenylene chain, subjecting another compound of general formula (I) where Z is a $C_{2-4}$ alkenylene or $C_{2-4}$ alkynylene chain to catalytic hydrogenation; or

A(vi) in order to prepare a compound of general formula (I) in which Y represents the group —A, —ZA or $OZ^1A$ where Z and $Z^1$ are as defined in claim 1 and A is a N-5-1H-tetrazolyl-carboxamide, reacting another compound of general formula (I) where A represents a carboxylic acid group with 5-aminotetrazole in the presence of a coupling agent; and

B(i) if desired reacting the resulting free acid of general formula (I) with a base to form a physiologically acceptable salt of the compound of general formula (I).

## Claims for the Contracting State: AT

1. A process for the preparation of a compound of general formula (I)

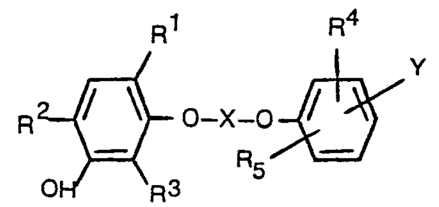

(I)

wherein

Y represents the group —A, —ZA or $OZ^1A$ wherein A represents a carboxylic acid group, a 5-1H-tetrazolyl ring, or N-5-1H-tetrazolylcarboxamide group;

Z represents a $C_{1-4}$ alkylene, $C_{2-4}$ alkenylene or $C_{2-4}$ alkynylene chain optionally substituted by one or more $C_{1-3}$ alkyl groups and $Z^1$ represents a $C_{1-4}$ alkylene chain optionally substituted by one or more $C_{1-3}$ alkyl groups:

X represents a $C_{1-10}$ carbon chain which may be saturated or unsaturated in the case of chains containing at least 4 carbon atoms, and may be substituted by a hydroxy group or by one or more $C_{1-3}$ alkyl groups, which chain may be interrupted by a benzene ring which may be linked through its 1- and 2-, 1- and 3-, or 1- and 4-positions;

$R^1$ represents a hydrogen atom or a $C_{1-6}$ alkyl group;

$R^2$ represents the group —$COR^6$ where $R^6$ represents a hydrogen atom, an aryl group, or a $C_{1-6}$ alkyl group which may be substituted by an aryl group;

$R^3$ represents a $C_{1-6}$ alkyl group or a $C_{3-6}$ alkenyl group; and

$R^4$ and $R^5$, which may be the same or different, each represents a hydrogen atom, a halogen atom or a hydroxy, $C_{1-3}$ alkoxy, $C_{1-6}$ alkyl, $C_{3-6}$ alkenyl, $C_{1-3}$ alkanoyl, nitro or carboxylic acid group, with the proviso that $R^4$ and $R^5$ cannot both be nitro, alkanoyl or carboxylic acid groups, or a physiologically acceptable salt thereof which comprises the steps:

A(i) in order to prepare a compound of general formula (I), in which —Y represents the group —$CH=CHCO_2H$, subjecting a compound of general formula (III)

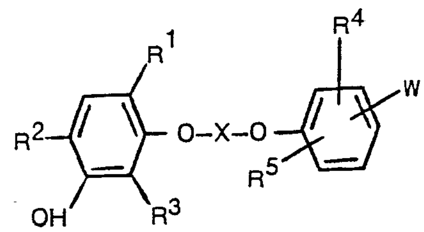

(III)

in which $R^1$ to $R^5$ and X are as defined above

W represents —CHO, to the Perkin synthesis of cinnamic acids; or

A(ii) in order to prepare a compound of general formula (I) in which Y represents the group —A, —ZA or —$OZ^1A$ where Z and $Z^1$ are as defined above and A is a carboxylic acid group, subjecting a compound of general formula (III) where W represents the group —E, —ZE or —$OZ^1E$ where E is a carboxylic ester, amide or nitrile group, to acid or base hydrolysis; or

A(iii) in order to prepare a compound of general formula (I) where Y represents the group —A, —ZA or —$OZ^1A$ where Z and $Z^1$ are as defined above and A is a 5-1H-tetrazolyl ring, reacting a compound of general formula (III) in which W represents —E, —ZE or $OZ^1E$ and E represents a nitrile, with sodium azide and ammonium chloride; or

A(iv) in order to prepare a compound of general formula (I) in which Y represents the group —A, —ZA or —$OZ^1A$ where A is a carboxylic acid group and Z and $Z^1$ are as defined above subjecting to hydrogenolysis a compound of general formula (III) in which W represents the group —E, —ZE or —$OZ^1E$ and E is a carboxylic ester group of formula —$COOR^7$ where $R^7$ is a group removable by hydrogenolysis; or

A(v) in order to prepare a compound of general formula (I) in which Y represents the group —ZA and Z is a $C_{2-4}$ alkylene or $C_{2-4}$ alkenylene chain, subjecting another compound of general formula (I) where Z is a $C_{2-4}$ alkenylene or $C_{2-4}$ alkynylene chain to catalytic hydrogenation; or

A(vi) in order to prepare a compound of general formula (I) in which Y represents the group —A, —ZA or —$OZ^1A$ where Z and $Z^1$ are as defined above and A is a N-5-1H-tetrazolyl-carboxamide, reacting another compound of general formula (I) where A represents a carboxylic acid group with 5-aminotetrazole in the presence of a coupling agent; and

B(i) if desired reacting the resulting free acid of general formula (I) with a base to form a physiologically acceptable salt of the compound of general formula (I).

2. A process according to claim 1 wherein the starting materials and reaction conditions are selected such that the product is a compound of general formula (I) wherein: Y represents a carboxylic acid group or a 5-1H-tetrazolyl ring or the group ZA or $OZ^1A$ where A is a carboxylic acid group and Z is a $C_{1-4}$ alkylene chain, a $C_{2-4}$ alkenylene chain or a $C_{2-4}$ alkynylene chain and $Z^1$ is methylene.

3. A process according to claim 1 wherein the starting materials and reaction conditions are selected such that the product is a compound of general formula (I) wherein: X represents a straight carbon chain of formula —$(CH_2)_n$ where *n* is a number from 1 to 10, or a group of formula —$CH_2X^1CH_2$— where X is a benzene ring linked through its 1- and 2-, 1- and 3- or 1- and 4-positions.

4. A process according to claim 1 wherein the starting materials and reaction conditions are selected such that the product is a compound of general formula (I) wherein: $R^1$ represents a hydrogen atom or a $C_{3-6}$ alkyl group.

5. A process according to claim 1 wherein the starting materials and reaction conditions are selected such that the product is a compound of general formula (I) wherein: $R^2$ represents the group $COR^6$ where $R^6$ represents a $C_{1-6}$ alkyl group.

6. A process according to claim 1 wherein the starting materials and reaction conditions are selected such that the product is a compound of general formula (I) wherein: $R^3$ represents a $C_{3-6}$ alkyl or $C_{3-6}$ alkenyl group.

7. A process according to claim 1 wherein the starting materials and reaction conditions are selected such that the product is a compound of general formula (I) wherein: $R^4$ represents a hydrogen or halogen atom or a hydroxy, nitro or $C_{1-3}$ alkanoyl group.

8. A process according to claim 1 wherein the starting materials and reaction conditions are selected such that the product is a compound of general formula (I) wherein: $R^5$ represents a hydrogen atom or a $C_{1-6}$ alkyl group or a $C_{2-4}$ alkenyl group.

9. A process according to claim 1 wherein the starting materials and reaction conditions are selected such that the product is a compound of general formula (I), wherein, $R^4$ represents a hydrogen atom or a hydroxyl group and $R^5$ is a hydrogen atom.

10. A process according to claim 1 wherein the starting materials and reaction conditions are selected such that the product is a compound of general formula (II)

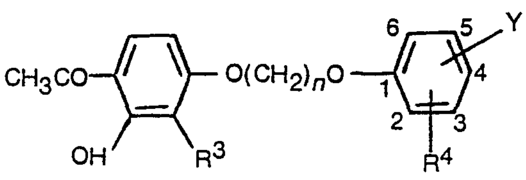

(II)

wherein Y is a carboxylic acid group or a 5-1H-tetrazolyl ring or a N-5-1H-tetrazolylcarboxamide group, or a $C_{1-2}$ alkyl, $C_{1-2}$ alkoxy or $C_2$-alkenyl chain terminally substituted by a carboxylic acid group and is situated at the 3- or 4-position in the benzene ring;

*n* is 1, 3 or 5;

$R_3$ is a $C_{3-6}$ alkyl or $C_{3-6}$ alkenyl group, and

$R^4$ is a hydrogen or bromine atom, or a hydroxy, nitro or acetyl group,

or a physiologically acceptable salt thereof.

11. A process according to claim 1 wherein the starting materials and reaction conditions are selected such that the product is a compound of general formula (II) wherein *n* is 3.

12. A process according to claim 1 wherein the starting materials and reaction conditions are selected such that the product is selected from 4-[3-(4-acetyl-3-hydroxy-2-propylphenoxy)propoxy]-2-hydroxybenzoic acid; 4-[3-(4-acetyl-3-hydroxy-2-propylphenoxy)propoxy]-2-hydroxy-N-1H-tetrazol-5-y-benzamide; 3-[3-[3-(4-acetyl-3-hydroxy-2-methylphenoxy)propoxy]phenyl]-2-propenoic acid; and physiologically acceptable salts of these compounds.

**Revendications pour les Etats contractants: BE CH DE FR GB IT LI NL SE**

1. Composé de formule générale (I)

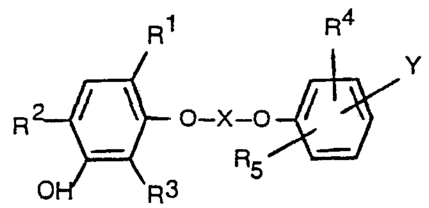

(I)

dans laquelle Y représente le groupe —A, —ZA ou —OZ$^1$A, dans lequel A représente un groupe acide carboxylique, un cycle 1H-tétrazolyle-5 ou un groupe N-1H-tétrazolyl-5 carboxamide, Z représente une chaîne alkylène en $C_{1-4}$, alcénylène en $C_{2-4}$ ou alcynylène en $C_{2-4}$ éventuellement substituée par un ou plusieurs groupes alkyle en $C_{1-3}$ et $Z^1$ représente un chaîne alkylène en $C_{1-4}$ éventuellement substituée par un ou plusieurs groupes alkyle en $C_{1-3}$; X représente une chaîne carbonée en $C_{1-10}$ qui peut être saturée ou insaturée dans le cas de chaînes contenant au moins 4 atomes de carbone et peut être substituée par un groupe hydroxyle ou par un ou plusieurs groupes alkyle en $C_{1-3}$, cette chaîne pouvant être interrompue par un cycle benzénique qui peut être lié par ses positions 1 et 2, 1 et 3, ou 1 et 4; $R^1$ représente un atome d'hydrogène ou un groupe alkyle en $C_{1-6}$; $R^2$ représente le groupe —$COR^6$ dans lequel $R^6$ représente un atome d'hydrogène, un groupe aryle ou un groupe alkyle en $C_{1-6}$ qui peut être substitue par un groupe aryle; $R^3$ représente un groupe alkyle en $C_{1-6}$ ou un groupe alcényle en $C_{3-6}$; et $R^4$ et $R^5$, qui peuvent être identiques ou différents, représentent chacun un atome d'hydrogène, un atome d'halogène ou un groupe hydroxy, alcoxy en $C_{1-3}$, alkyle en $C_{1-6}$, alcényle en $C_{3-6}$, alcanoyle en $C_{1-3}$, nitro ou acide carboxylique, à condition que $R^4$ et $R^5$ ne soient pas ensemble des groupes nitro, alcanoyle ou acide carboxylique, et ses sels physiologiquement acceptables.

2. Composé selon la revendication 1, caractérisé en ce que, dans la formule générale (I), Y représente un groupe acide carboxylique ou un cycle 1H-tétrazolyle-5 ou le groupe ZA ou OZ$^1$A dans lequel A est un groupe acide carboxylique et Z est une chaîne alkylène en $C_{1-4}$, une chaîne alcénylène en $C_{2-4}$ ou une chaîne alcynylène en $C_{2-4}$ et Z est le méthylène.

3. Composé selon la revendication 1 ou 2, caractérisé en ce que, dans la formule générale (I), X représente une chaîne carbonée droite de formule —$(CH_2)_n$ dans laquelle *n* est un nombre de 1 à 10, ou un groupe de formule —$CH_2X^1CH_2$— dans laquelle $X^1$ est un cycle benzénique lié par ses positions 1 et 2, 1 et 3 ou 1 et 4.

4. Composé selon l'une quelconque des revendications 1 à 3, caractérisé en ce que, dans la formule générale (I), $R^1$ représente un atome d'hydrogène ou un groupe alkyle en $C_{3-6}$.

5. Composé selon l'une quelconque des revendications 1 à 4, caractérisé en ce que, dans la formule générale (I), $R^2$ représente le groupe $COR^6$ où $R^6$ représente un groupe alkyle en $C_{1-6}$.

6. Composé selon l'une quelconque des revendications 1 à 5, caractérisé en ce que, dans la formule générale (I), $R^3$ représente un groupe alkyle en $C_{3-6}$ ou un groupe alcényle en $C_{3-6}$.

7. Composé selon l'une quelconque des revendications 1 à 6, caractérisé en ce que, dans la formule générale (I), $R^4$ représente un atome d'hydrogène ou d'halogène ou un groupe hydroxy, nitro ou alcanoyle en $C_{1-3}$.

8. Composé selon l'une quelconque des revendications 1 à 7, caractérisé en ce que, dans la formule générale (I), $R^5$ représente un atome d'hydrogène ou un groupe alkyle en $C_{1-6}$ ou un groupe alcényle en $C_{2-4}$.

9. Composé selon l'une quelconque des revendications 1 à 8, caractérisé en ce que, dans la formule générale (I), $R^4$ représente un atome d'hydrogène ou un groupe hydroxyle et $R^5$ est un atome d'hydrogène.

10. Composé de formule générale (II)

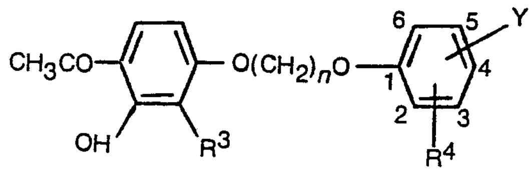

(II)

dans laquelle Y est un groupe acide carboxylique ou un cycle 1H-tétrazolyle-5 ou un groupe N-1H-tétrazolyl-5 carboxamide ou une chaîne alkyle en $C_{1-2}$, alcoxy en $C_{1-2}$ ou alcényle en $C_2$ substitué en bout par un groupe acide carboxylique, $R^3$ représente un groupe alkyle en $C_{3-6}$ ou un groupe alcényle en $C_{3-6}$, $R^4$ représente un atome d'hydrogène ou de brome ou un groupe hydroxy, nitro ou acétyle et *n* est 1, 3 ou 5 et ses sels physiologiquement acceptables.

11. Composé selon la revendication 10, caractérisé en ce que, dans la formule générale (II), Y est situé en position 3 ou 4 du cycle benzénique.

12. Composé selon la revendication 10 ou 11, caractérisé en ce que, dans la formule générale (II), *n* est 3.

13. L'acide [(acétyl-4 hydroxy-3 propyl-2 phénoxy)-3 propoxy]-4 hydroxy-2 benzoïque et ses sels physiologiquement acceptables.

14. L'[(acétyl-4 hydroxy-3 propyl-2 phénoxy)-3 propoxy]-4 hydroxy-2 N-1H-tétrazolyl-5 benzamide et ses sels physiologiquement acceptables.

15. L'acide [[(acétyl-4 hydroxy-3 méthyl-2 phénoxy)-3 propoxy]-3 phényl]-3 propène-2 oïque et ses sels physiologiquement acceptables.

16. Composition pharmaceutique comprenant, comme principe actif, un ou plusieurs composés selon l'une quelconque des revendications 1 à 15, avec un ou plusieurs de ses véhicules ou excipients physiologiquement acceptables.

17. Composition pharmaceutique selon la revendication 16, caractérisée en ce qu'elle est formulée en poudre sous une forme convenable pour l'administration par inhalation.

18. Procédé de préparation d'un composé de formule générale (I) tel que défini dans la revendication 1 ou d'un de ses sels physiologiquement acceptable caractérisé en ce qu'il comprend les étapes de:

A(i) préparation d'un composé de formule générale (I), dans laquelle —Y représente le groupe —$CH=CHCO_2H$, en soumettant un composé de formule générale (III)

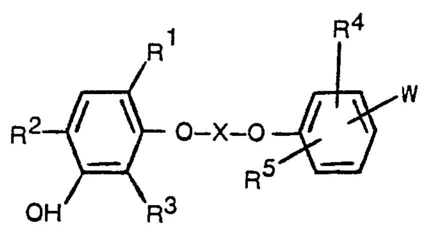

(III)

dans laquelle $R^1$ à $R^5$ et X sont tels que définis dans la revendication 1 et W représente —CHO, à la synthèse de Perkin des acides cinnamiques; ou

A(ii) préparation d'un composé de formule générale (I), dans laquelle Y représente le groupe —A, —ZA ou —$OZ^1$A dans lequel Z et $Z^1$ sont tels que définis dans la revendication 1 et A est un groupe acide carboxylique, en soumettant un composé de formule générale (III), dans laquelle W représente le groupe —E, —ZE ou —$OZ^1$E où E est un groupe ester carboxylique, amide ou nitrile, à une hydrolyse acide ou basique; ou

A(iii) préparation d'un composé de formule générale (I), dans laquelle Y représente le groupe —A, —ZA ou —$OZ^1$A dans lequel Z et $Z^1$ sont tels que définis dans la revendication 1 et A est un cycle 1H-tétrazolyle-5, en faisant réagir un composé de formule générale (III), dans laquelle W représente —E, —ZE ou —$OZ^1$E et E représente un nitrile, avec l'azoture de sodium et le chlorure d'ammonium; ou

A(iv) préparation d'un composé de formule générale (I), dans laquelle Y représente le groupe —A, —ZA ou —$OZ^1$A dans lequel A est un groupe acide carboxylique et Z et $Z^1$ sont tels que définis dans la revendication 1, en soumettant un composé de formule générale (III), dans laquelle W représente le groupe —E, —ZE ou —$OZ^1$E et E est un groupe ester carboxylique de formule —$COOR^7$ où $R^7$ est un groupe pouvant être retiré par hydrogénolyse, à une hydrogénolyse; ou

A(v) préparation d'un composé de formule générale (I), dans laquelle Y représente le groupe —ZA dans lequel Z est une chaîne alkylène en $C_{2-4}$ ou alcénylène en $C_{2-4}$, en soumettant un autre composé de formule générale (I), dans laquelle Z est une chaîne alcénylène en $C_{2-4}$ ou alcynylène en $C_{2-4}$, à une hydrogénation catalytique; ou

A(vi) préparation d'un composé de formule générale (I), dans laquelle Y représente le groupe —A, —ZA ou —$OZ^1A$ dans lequel Z et $Z^1$ sont tels que définis dans la revendication 1 et A est un N-1H-tétrazolyl-5 carboxamide, en faisant réagir un autre composé de formule générale (I), dans laquelle A représente un groupe acide carboxylique, avec l'amino-5 tétrazole en présence d'un agent de couplage; et

B(i) réaction éventuelle de l'acide libre obtenu de formule générale (I) avec une base pour former un sel physiologiquement acceptable du composé de formule générale (I).

**Revendications pour l'Etat contractant: AT**

1. Procédé de préparation d'un composé de formule générale (I)

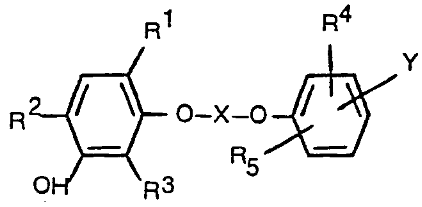

(I)

dans laquelle Y représente le groupe —A, —ZA ou —$OZ^1A$, dans lequel A représente un groupe acide carboxylique, un cycle 1H-tétrazolyle-5 ou un groupe N-1H-tétrazolyl-5 carboxamide, Z représente une chaîne alkylène en $C_{1-4}$, alcénylène en $C_{2-4}$ ou alcynylène en $C_{2-4}$ éventuellement substituée par un ou plusieurs groupes alkyle en $C_{1-3}$ et $Z^1$ représente une chaîne alkylène en $C_{1-4}$ éventuellement substituée par un ou plusieurs groupes alkyle en $C_{1-3}$; X représente une chaîne carbonée en $C_{1-10}$ qui peut être saturée ou insaturée dans le cas de chaînes contenant au moins 4 atomes de carbone et peut être substituée par un groupe hydroxyle ou par un ou plusieurs groupes alkyle en $C_{1-3}$, cette chaîne pouvant être interrompue par un cycle benzénique qui peut être lié par ses positions 1 et 2, 1 et 3, ou 1 et 4; $R^1$ représente un atome d'hydrogène ou un groupe alkyle en $C_{1-6}$; $R^2$ représente le groupe —$COR^6$ dans lequel $R^6$ représente un atome d'hydrogène, un groupe aryle ou un groupe alkyle en $C_{1-6}$ qui peut être substitue par un groupe aryle; $R^3$ représente un groupe alkyle en $C_{1-6}$ ou un groupe alcényle en $C_{3-6}$; et $R^4$ et $R^5$, qui peuvent être identiques ou différents, représentent chacun un atome d'hydrogène, un atome d'halogène ou un groupe hydroxy, alcoxy en $C_{1-3}$, alkyle en $C_{1-6}$, alcényle en $C_{3-6}$, alcanoyle en $C_{1-3}$, nitro ou acide carboxylique, à condition que $R^4$ et $R^5$ ne soient pas ensemble des groupes nitro, alcanoyle ou acide carboxylique, ou d'un de ses sels physiologiquement acceptable, caractérisé en ce qu'il comprend les étapes de:

A(i) préparation d'un composé de formule générale (I), dans laquelle —Y représente le groupe —$CH{=}CHCO_2H$, en soumettant un composé de formule générale (III)

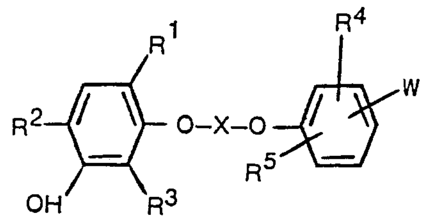

(III)

dans laquelle $R^1$ à $R^5$ et X sont tels que définis ci-dessus W représente —CHO, à la synthèse de Perkin des acides cinnamiques; ou

A(ii) préparation d'un composé de formule générale (I), dans laquelle Y représente le groupe —A, —ZA ou —$OZ^1A$ dans lequel Z et $Z^1$ sont tels que définis ci-dessus et A est un groupe acide carboxylique, en soumettant un composé de formule générale (III), dans laquelle W représente le groupe —E, —ZE ou —$OZ^1E$ où E est un groupe ester carboxylique, amide ou nitrile, à une hydrolyse acide ou basique; ou

A(iii) préparation d'un composé de formule générale (I), dans laquelle Y représente le groupe —A, —ZA ou —$OZ^1A$ dans lequel Z et $Z^1$ sont tels que définis ci-dessus et A est un cycle 1H-tétrazolyle-5, en faisant réagir un composé de formule générale (III), dans laquelle W représente —E, —ZE ou —$OZ^1E$ et E représente un nitrile, avec l'azoture de sodium et le chlorure d'ammonium; ou

A(iv) préparation d'un composé de formule générale (I), dans laquelle Y représente le groupe —A, —ZA ou —$OZ^1A$ dans lequel A est un groupe acide carboxylique et Z et $Z^1$ sont tels que définis ci-dessus, en soumettant un composé de formule générale (III), dans laquelle W représente le groupe —E, —ZE ou —$OZ^1E$ et E est un groupe ester carboxylique de formule —$COOR^7$ où $R^7$ est un groupe pouvant être retiré par hydrogénolyse, à une hydrogénolyse; ou

A(v) préparation d'un composé de formule générale (I), dans laquelle Y représente le groupe —ZA dans

lequel Z est une chaîne alkylène en $C_{2-4}$ ou alcénylène en $C_{2-4}$, en soumettant un autre composé de formule générale (I), dans laquelle Z est une chaîne alcénylène en $C_{2-4}$ ou alcynylène en $C_{2-4}$, à une hydrogénation catalytique; ou

A(vi) préparation d'un composé de formule générale (I), dans laquelle Y représente le groupe —A, —ZA ou —OZ¹A dans lequel Z et Z¹ sont tels que définis ci-dessus et A est un N-1H-tétrazolyl-5 carboxamide, en faisant réagir un autre composé de formule générale (I), dans laquelle A représente un groupe acide carboxylique, avec l'amino-5 tétrazole en présence d'un agent de couplage; et

B(i) réaction éventuelle de l'acide libre obtenu de formule générale (I) avec une base pour former un sel physiologiquement acceptable du composé de formule générale (I).

2. Procédé selon la revendication 1, caractérisé en ce que les matériaux de départ et les conditions réactionnelles sont choisies de telle sorte que le produit soit un composé de formule générale (I) dans laquelle Y représente un groupe acide carboxylique ou un cycle 1H-tétrazolyle-5 ou le groupe ZA ou OZ¹A dans lequel A est un groupe acide carboxylique et Z est une chaîne alkylène en $C_{1-4}$, une chaîne alcénylène en $C_{2-4}$ ou une chaîne alcynylène en $C_{2-4}$ et Z est le méthylène.

3. Procédé selon la revendication 1, caractérisé en ce que les matériaux de départ et les conditions réactionnelles sont choisies de telle sorte que le produit soit un composé de formule générale (I) dans laquelle X représente une chaîne carbonée droite de formule $—(CH_2)_n$ dans laquelle *n* est un nombre de 1 à 10, ou un groupe de formule $—CH_2X^1CH_2—$ dans laquelle $X^1$ est un cycle benzénique lié par ses positions 1 et 2, 1 et 3 ou 1 et 4.

4. Procédé selon la revendication 1, caractérisé en ce que les matériaux de départ et les conditions réactionnelles sont choisies de telle sorte que le produit soit un composé de formule générale (I) dans laquelle $R^1$ représente un atome d'hydrogène ou un groupe alkyle en $C_{3-6}$.

5. Procédé selon la revendication 1, caractérisé en ce que les matériaux de départ et les conditions réactionnelles sont choisies de telle sorte que le produit soit un composé de formule générale (I) dans laquelle $R^2$ représente le groupe $COR^6$ où $R^6$ représente un groupe alkyle en $C_{1-6}$.

6. Procédé selon la revendication 1, caractérisé en ce que les matériaux de départ et les conditions réactionnelles sont choisies de telle sorte que le produit soit un composé de formule générale (I) dans laquelle $R^3$ représente un groupe alkyle en $C_{3-6}$ ou un groupe alcényle en $C_{3-6}$.

7. Procédé selon la revendication 1, caractérisé en ce que les matériaux de départ et les conditions réactionnelles sont choisies de telle sorte que le produit soit un composé de formule générale (I) dans laquelle $R^4$ représente un atome d'hydrogène ou d'halogène ou un groupe hydroxy, nitro ou alcanoyle en $C_{1-3}$.

8. Procédé selon la revendication 1, caractérisé en ce que les matériaux de départ et les conditions réactionnelles sont choisies de telle sorte que le produit soit un composé de formule générale (I) dans laquelle $R^5$ représente un atome d'hydrogène ou un groupe alkyle en $C_{1-6}$ ou un groupe alcényle en $C_{2-4}$.

9. Procédé selon la revendication 1, caractérisé en ce que les matériaux de départ et les conditions réactionnelles sont choisies de telle sorte que le produit soit un composé de formule générale (I) dans laquelle $R^4$ représente un atome d'hydrogène ou un groupe hydroxyle et $R^5$ est un atome d'hydrogène.

10. Procédé selon la revendication 1, caractérisé en ce que les matériaux de départ et les conditions réactionnelles sont choisies de telle sorte que le produit soit un composé de formule générale (II)

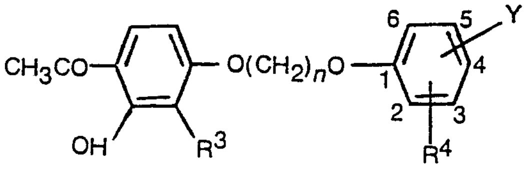

(II)

dans laquelle Y est un groupe acide carboxylique ou un cycle 1H-tétrazolyle-5 ou un groupe N-1H-tétrazolyl-5 carboxamide ou une chaîne alkyle en $C_{1-2}$, alcoxy en $C_{1-2}$, ou alcényle en $C_2$ substituée en bout par un groupe acide carboxylique et est situé en position 3 ou 4 du cycle benzénique, *n* est 1, 3 ou 5, $R^3$ est un groupe alkyle en $C_{3-6}$ ou un groupe alcényle en $C_{3-6}$ et $R^4$ est un atome d'hydrogène ou de brome, ou un groupe hydroxy, nitro ou acétyle, ou un de ses sels physiologiquement acceptables.

11. Procédé selon la revendication 1, caractérisé en ce que les matériaux de départ et les conditions réactionnelles sont choisies de telle sorte que le produit soit un composé de formule générale (II) dans laquelle *n* est 3.

12. Procédé selon la revendication 1, caractérisé en ce que les matériaux de départ et les conditions réactionnelles sont choisies de telle sorte que le produit soit choisi parmi l'acide [(acétyl-4 hydroxy-3 propyl-2 phénoxy)-3 propoxy]-4 hydroxy-2 benzoïque, 1' [(acétyl-4 hydroxy-3 propyl-2 phénoxy)-3 propoxy]-4 hydroxy-2 N-1H-tétrazolyl-5 benzamide, l'acide [[(acétyl-4 hydroxy-3 méthyl-2 phénoxy-3 propoxy]-3 phényl]-3 propène-2 oïque et les sels physiologiquement acceptables de ces composés.

**Patentansprüche für die Vertragsstaaten: BE CH DE FR GB IT LI NL SE**

1. Verbindungen der allgemeinen Formel (I):

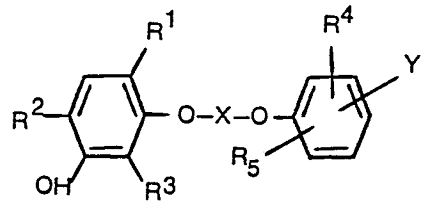

(I)

in der Y für die Gruppe —A, —ZA oder $OZ^1A$ steht, wobei A für eine Carbonsäuregruppe, eine 5-1H-Tetrazolylring oder eine N-5-1H-Tetrazolylcarboxamidgruppe steht, Z für eine $C_{1-4}$-Alkylen-, $C_{2-4}$-Alkenylen- oder $C_{2-4}$-Alkinylenkette, die gegebenenfalls durch eine oder mehrere $C_{1-3}$-Alkylgruppen substituiert ist, steht und $Z^1$ für eine $C_{1-4}$-Alkylenkette, die gegebenenfalls durch eine oder mehrere $C_{1-3}$-Alkylgruppen substituiert ist, steht, X eine $C_{1-10}$-Kohlenstoffkette bedeutet, die gesättigt sein kann oder im Falle von Ketten, die mindestens 4 Kohlenstoffatome enthalten, ungesättigt sein kann und die durch eine Hydroxylgruppe oder durch eine oder mehrere $C_{1-3}$-Alkylgruppen substituiert sein kann, welche Kette durch einen Benzolring unterbrochen sein kann, der damit durch seine 1- und 2-, 1- und 3- oder 1- une 4-Stellungen verbunden sein kann, $R^1$ für ein Wasserstoffatom oder eine $C_{1-6}$-Alkylgruppe steht, $R^2$ für die Gruppe $—COR^6$ steht, wobei $R^6$ ein Wasserstoffatom, eine Arylgruppe oder eine $C_{1-6}$-Alkylgruppe, die durch eine Arylgruppe substituiert sein kann, steht, $R^3$ für eine $C_{1-6}$-Alkylgruppe oder eine $C_{3-6}$-Alkenylgruppe steht und $R^4$ und $R^5$, die gleich oder verschieden sein können, jeweils für ein Wasserstoffatom, ein Halogenatom oder eine Hydroxy-, $C_{1-3}$-Alkoxy-, $C_{1-6}$-Alkyl-, $C_{3-6}$-Alkenyl-, $C_{1-3}$-Alkanoyl-, Nitro- oder Carbonsäuregruppe stehen, mit der Maßgabe, daß beide Gruppen $R^4$ und $R^5$ nicht Nitro-, Alkanoyl- oder Carbonsäuregruppen sein können, und die physiologisch annehmbaren Salze davon.

2. Verbindungen nach Anspruch 1, dadurch gekennzeichet, daß in der allgemeinen Formel (I) Y für eine Carbonsäuregruppe oder einen 5-1H-Tetrazolylring oder die Gruppe ZA oder $OZ^1A$ steht, wobei A eine Carbonsäuregruppe ist und Z eine $C_{1-4}$-Alkylenkette, eine $C_{2-4}$-Alkenylenkette oder ein $C_{2-4}$-Alkinylenkette ist und $Z^1$ Methylen ist.

3. Verbindungen nach Anspruch 1 oder 2, dadurch gekennzeichnet, daß in der allgemeinen Formel (I) X für eine geradkettige Kohlenstoffkette der Formel $—(CH_2)_n$, wobei n eine Zahl von 1 bis 10 ist, oder eine Gruppe der Formel $—CH_2X^1CH_2—$, wobei X ein damit durch seine 1- und 2-, 1- und 3- oder 1- und 4-Stellungen verbundener Benzolring ist, steht.

4. Verbindungen nach einem der Ansprüche 1 bis 3, dadurch gekennzeichnet, daß in der allgemeinen Formel (I) $R^1$ für ein Wasserstoffatom oder eine $C_{3-6}$-Alkylgruppe steht.

5. Verbindungen nach einem der Ansprüche 1 bis 4, dadurch gekennzeichnet, daß in der allgemeinen Formel (I) $R^2$ für die Gruppe $COR^6$ steht, wobei $R^6$ für eine $C_{1-6}$-Alkylgruppe steht.

6. Verbindungen nach einem der Ansprüche 1 bis 5, dadurch gekennzeichnet, daß in der allgemeinen Formel (I) $R^3$ für eine $C_{3-6}$-Alkyl- oder $C_{3-6}$-Alkenylgruppe steht.

7. Verbindungen nach einem der Ansprüche 1 bis 6, dadurch gekennzeichnet, daß in der allgemeinen Formel (I) $R^4$ für ein Wasserstoff- oder Halogenatom oder eine Hydroxy-, Nitro- oder $C_{1-3}$-Alkanoylgruppe steht.

8. Verbindungen nach einem der Ansprüche 1 bis 7, dadurch gekennzeichnet, daß in der allgemeinen Formel (I) $R^5$ für ein Wasserstoffatom oder eine $C_{1-6}$-Alkylgruppe oder eine $C_{2-4}$-Alkenylgruppe steht.

9. Verbindungen nach einem der Ansprüche 1 bis 8, dadurch gekennzeichnet, daß in der allgemeinen Formel (I) $R^4$ für ein Wasserstoffatom oder eine Hydroxylgruppe steht und $R^5$ für ein Wasserstoffatom steht.

10. Verbindungen der allgemeinen Formel (II):

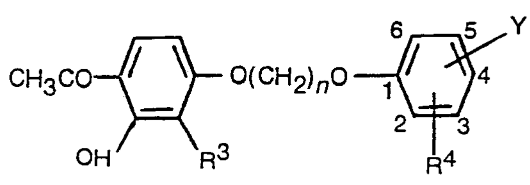

(II)

in der Y für eine Carbonsäuregruppe oder einen 5-1H-Tetrazolylring oder eine N-5-1H-Tetrazolylcarboxamidgruppe oder eine $C_{1-2}$-Alkyl-, $C_{1-2}$-Alkoxy- oder $C_2$-Alkenylkette, die endständig durch eine Carbonsäuregruppe substituiert ist, steht, $R^3$ für eine $C_{3-6}$-Alkyl- oder $C_{3-6}$-Alkenylgruppe steht, $R^4$ für

ein Wasserstoff- oder Bromatom oder eine Hydroxy-, Nitro- oder Acetylgruppe steht und n 1, 3 oder 5 ist, und die physiologisch annehmbaren Salze davon.

11. Verbindung nach Anspruch 10, dadurch gekennzeichnet, daß in der allgemeinen Formel (II) Y an der 3- oder 4-Stellung des Benzolrings angeordnet ist.

12. Verbindung nach Anspruch 10 oder 11, dadurch gekennzeichnet, daß in der allgemeinen Formel (II) n den Wert 3 hat.

13. 4-[3-(4-Acetyl-3-hydroxy-2-propylphenoxy)-propoxy]-2-hydroxybenzoesäure und ihre physiologisch annehmbaren Salze.

14. 4-[3-(4-Acetyl-3-hydroxy-2-propylphenoxy)-propoxy]-2-hydroxy-N-1H-tetrazol-5-y-benzamid und seine physiologisch annehmbaren Salze.

15. 3-[3-[3-(4-Acetyl-3-hydroxy-2-methylphenoxy)-propoxy]-phenyl]-2-propensäure und ihre physiologisch annehmbaren Salze.

16. Arzneimittel, dadurch gekennzeichnet, daß es als Wirkstoff eine oder mehrere Verbindungen nach einem der Ansprüche 1 bis 15 zusammen mit einem oder mehreren physiologisch annehmbaren Trägern oder Streckmitteln hierfür enthält.

17. Arzneimittel nach Anspruch 16, dadurch gekennzeichnet, daß es als Pulver in einer für die Verabreichung durch Inhalierung geeigneten Form formuliert ist.

18. Verfahren zur Herstellung einer Verbindung der allgemeinen Formel (I) nach Anspruch 1 oder eines physiologisch annehmbaren Salzes davon, gekennzeichnet durch die Stufen:

A(i) daß man zur Herstellung einer Verbindung der allgemeinen Formel (I), in der Y die Gruppe —$CH{=}CHCO_2H$ ist, eine Verbindung der allgemeinen Formel (III):

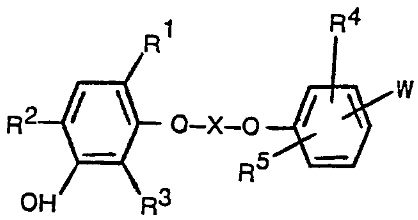

(III)

worin $R^1$ bis $R^5$ und X wie im Anspruch 1 definiert sind und W für —CHO steht, der Perkin'schen Synthese von Zimtsäuren unterwirft, oder

A(ii) daß man zur Herstellung einer Verbindung der allgemeinen Formel (I), bei der Y die Gruppe —A, —ZA oder —$OZ^1A$ ist, wobei Z und $Z^1$ wie im Anspruch 1 definiert sind und A eine Carbonsäuregruppe ist, eine Verbindung der allgemeinen Formel (III), wobei W für die Gruppe —E, —ZE oder —$OZ^1E$ steht, wobei E eine Carbonsäureester-, Amid- oder Nitrilgruppe ist, einer sauren oder basischen Hydrolyse unterwirft, oder

A(iii) daß man zur Herstellung einer Verbindung der allgemeinen Formel (I), bei der Y die Gruppe —A, —ZA oder —$OZ^1A$ ist, wobei Z und $Z^1$ wie im Anspruch 1 definiert sind und A ein 5-1H-Tetrazolylring ist, eine Verbindung der allgemeinen Formel (III), worin W für —E, —ZE oder —$OZ^1E$ steht und E ein Nitril bedeutet, mit Natriumazid und Ammoniumchlorid umsetzt, oder

A(iv) daß man zur Herstellung einer Verbindung der allgemeinen Formel (I), bei der Y die Gruppe —A, —ZA oder —$OZ^1A$ ist, wobei A eine Carbonsäuregruppe ist und Z und $Z^1$ wie im Anspruch 1 definiert sind, eine Verbindung der allgemeinen Formel (III), worin W die Gruppe —E, —ZE oder —$OZ^1E$ ist und E eine Carbonsäureestergruppe der Formel —$COOR^7$ ist, wobei $R^7$ eine durch Hydrogenolyse entfernbare Gruppe ist, einer Hydrogenolyse unterwirft, oder

A(v) daß man zur Herstellung einer Verbindung der allgemeinen Formel (I), bei der Y die Gruppe —ZA ist, wobei Z eine $C_{2-4}$-Alkylen- oder $C_{2-4}$-Alkenylenkette ist, eine andere Verbindung der allgemeinen Formel (I), bei der Z eine $C_{2-4}$-Alkenylen- oder $C_{2-4}$-Alkinylenkette ist, einer katalytischen Hydrierung unterwirft, oder

A(vi) daß man zur Herstellung einer Verbindung der allgemeinen Formel (I), bei der Y die Gruppe —A, —ZA oder —$OZ^1A$ ist, wobei Z und $Z^1$ wie im Anspruch 1 definiert sind und A ein N-5-1H-Tetrazolylcarboxamid ist, eine andere Verbindung der allgemeinen Formel (I), bei der A eine Carbonsäuregruppe ist, mit 5-Aminotetrazol in Gegenwart eines Kupplungsmittels umsetzt, und

B(i) gewünschtenfalls die resultierende freie Säure der allgemeinen Formel (I) mit einer Base unter Bildung eines physiologisch annehmbaren Salzes der Verbindung der allgemeinen Formel (I) umsetzt.

**Patentansprüche für den Vertragsstaat: AT**

1. Verfahren zur Herstellung einer Verbindung der allgemeinen Formel (I):

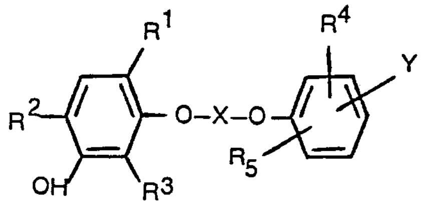

(I)

in der Y für die Gruppe —A, —ZA oder $OZ^1A$ steht, wobei A für eine Carbonsäuregruppe, eine 5-1H-Tetrazolylring oder eine N-5-1H-Tetrazolylcarboxamidgruppe steht, Z für eine $C_{1-4}$-Alkylen-, $C_{2-4}$-Alkenylen- oder $C_{2-4}$-Alkinylenkette, die gegebenenfalls durch eine oder mehrere $C_{1-3}$-Alkylgruppen substituiert ist, steht und $Z^1$ für eine $C_{1-4}$-Alkylenkette, die gegebenenfalls durch eine oder mehrere $C_{1-3}$-Alkylgruppen substituiert ist, steht, X eine $C_{1-10}$-Kohlenstoffkette bedeutet, die gesättigt sein kann oder im Falle von Ketten, die mindestens 4 Kohlenstoffatome enthalten, ungesättigt sein kann und die durch eine Hydroxylgruppe oder durch eine oder mehrere $C_{1-3}$-Alkylgruppen substituiert sein kann, welche Kette durch einen Benzolring unterbrochen sein kann, der damit durch seine 1- und 2-, 1- und 3- oder 1- une 4-Stellungen verbunden sein kann, $R^1$ für ein Wasserstoffatom oder eine $C_{1-6}$-Alkylgruppe steht, $R^2$ für die Gruppe —$COR^6$ steht, wobei $R^6$ ein Wasserstoffatom, eine Arylgruppe oder eine $C_{1-6}$-Alkylgruppe, die durch eine Arylgruppe substituiert sein kann, steht, $R^3$ für eine $C_{1-6}$-Alkylgruppe oder eine $C_{3-6}$-Alkenylgruppe steht und $R^4$ und $R^5$, die gleich oder verschieden sein können, jeweils für ein Wasserstoffatom, ein Halogenatom oder eine Hydroxy-, $C_{1-3}$-Alkoxy-, $C_{1-6}$-Alkyl-, $C_{3-6}$-Alkenyl-, $C_{1-3}$-Alkanoyl-, Nitro- oder Carbonsäuregruppe stehen, mit der Maßgabe, daß beide Gruppen $R^4$ und $R^5$ nicht Nitro-, Alkanoyl- oder Carbonsäuregruppen sein können, oder eines physiologisch annehmbaren Salzes davon, gekennzeichnet durch die Stufen:

A(i) daß man zur Herstellung einer Verbindung der allgemeinen Formel (I), in der Y die Gruppe —$CH{=}CHCO_2H$ ist, eine Verbindung der allgemeinen Formel (III):

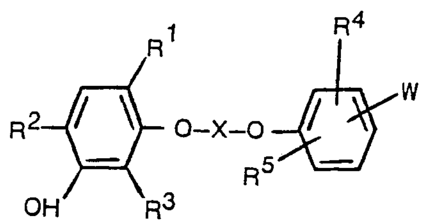

(III)

worin $R^1$ bis $R^5$ und X wie vorstehend definiert sind und W für —CHO steht, der Perkin'schen Synthese von Zimtsäuren unterwirft, oder

A(ii) daß man zur Herstellung einer Verbindung der allgemeinen Formel (I), bei der Y die Gruppe —A, —ZA oder —$OZ^1A$ ist, wobei Z und $Z^1$ wie vorstehend definiert sind und A eine Carbonsäuregruppe ist, eine Verbindung der allgemeinen Formel (III), wobei W für die Gruppe —E, —ZE oder —$OZ^1E$ steht, wobei E eine Carbonsäureester-, Amid- oder Nitrilgruppe ist, einer sauren oder basischen Hydrolyse unterwirft, oder

A(iii) daß man zur Herstellung einer Verbindung der allgemeinen Formel (I), bei der Y die Gruppe —A, —ZA oder —$OZ^1A$ ist, wobei Z und $Z^1$ wie vorstehend definiert sind und A ein 5-1H-Tetrazolylring ist, eine Verbindung der allgemeinen Formel (III), worin W für —E, —ZE oder —$OZ^1E$ steht und E ein Nitril bedeutet, mit Natriumazid und Ammoniumchlorid umsetzt, oder

A(iv) daß man zur Herstellung einer Verbindung der allgemeinen Formel (I), bei der Y die Gruppe —A, —ZA oder —$OZ^1A$ ist, wobei A eine Carbonsäuregruppe ist und Z und $Z^1$ wie vorstehend definiert sind, eine Verbindung der allgemeinen Formel (III), worin W die Gruppe —E, —ZE oder —$OZ^1E$ ist und E eine Carbonsäureestergruppe der Formel —$COOR^7$ ist, wobei $R^7$ eine durch Hydrogenolyse entfernbare Gruppe ist, einer Hydrogenolyse unterwirft, oder

A(v) daß man zur Herstellung einer Verbindung der allgemeinen Formel (I), bei der Y die Gruppe —ZA ist, wobei Z eine $C_{2-4}$-Alkylen- oder $C_{2-4}$-Alkenylenkette ist, eine andere Verbindung der allgemeinen Formel (I), bei der Z eine $C_{2-4}$-Alkenylen- oder $C_{2-4}$-Alkinylenkette ist, einer katalytischen Hydrierung unterwirft, oder

A(vi) daß man zur Herstellung einer Verbindung der allgemeinen Formel (I), bei der Y die Gruppe —A, —ZA oder —$OZ^1A$ ist, wobei Z und $Z^1$ wie vorstehend definiert sind und A ein N-5-1H-Tetrazolylcarboxamid ist, eine andere Verbindung der allgemeinen Formel (I), bei der A eine Carbonsäuregruppe

ist, mit 5-Aminotetrazol in Gegenwart eines Kupplungsmittels umsetzt, und
B(i) gewünschtenfalls die resultierende freie Säure der allgemeinen Formel (I) mit einer Base unter Bildung eines physiologisch annehmbaren Salzes der Verbindung der allgemeinen Formel (I) umsetzt.

2. Verfahren nach Anspruch 1, dadurch gekennzeichet, daß man die Ausgangsmaterialien und die Reaktionsbedingungen so auswählt, daß das Produkt eine Verbindung der allgemeinen Formel (I) ist, die dadurch gekennzeichnet ist, daß Y für eine Carbonsäuregruppe oder einen 5-1H-Tetrazolylring oder die Gruppe ZA oder $OZ^1A$ steht, wobei A eine Carbonsäuregruppe ist und Z eine $C_{1-4}$-Alkylenkette, eine $C_{2-4}$-Alkenylenkette oder eine $C_{2-4}$-Alkinylenkette ist und $Z^1$ Methylen ist.

3. Verfahren nach Anspruch 1, dadurch gekennzeichnet, daß man die Ausgangsmaterialien und die Reaktionsbedingungen so auswählt, daß das Produkt eine Verbindung der allgemeinen Formel (I) ist, die dadurch gekennzeichnet ist, daß X für eine geradkettige Kohlenstoffkette der Formel $-(CH_2)_n$, wobei n eine Zahl von 1 bis 10 ist, oder eine Gruppe der Formel $-CH_2X^1CH_2-$, wobei X ein damit durch seine 1- und 2-, 1- und 3- oder 1- und 4-Stellungen verbundener Benzolring ist, steht.

4. Verfahren nach Anspruch 1, dadurch gekennzeichnet, daß man die Ausgangsmaterialien und die Reaktionsbedingungen so auswählt, daß das Produkt eine Verbindung der allgemeinen Formel (I) ist, die dadurch gekennzeichnet ist, daß $R^1$ für ein Wasserstoffatom oder eine $C_{3-6}$-Alkylgruppe steht.

5. Verfahren nach Anspruch 1, dadurch gekennzeichnet, daß man die Ausgangsmaterialien und die Reaktionsbedingungen so auswählt, daß das Produkt eine Verbindung der allgemeinen Formel (I) ist, die dadurch gekennzeichnet ist, daß $R^2$ für die Gruppe $COR^6$ steht, wobei $R^6$ für eine $C_{1-6}$-Alkylgruppe steht.

6. Verfahren nach Anspruch 1, dadurch gekennzeichnet, daß man die Ausgangsmaterialien und die Reaktionsbedingungen so auswählt, daß das Produkt eine Verbindung der allgemeinen Formel (I) ist, die dadurch gekennzeichnet ist, daß $R^3$ für eine $C_{3-6}$-Alkyl- oder $C_{3-6}$-Alkenylgruppe steht.

7. Verfahren nach Anspruch 1, dadurch gekennzeichnet, daß man die Ausgangsmaterialien und die Reaktionsbedingungen so auswählt, daß das Produkt eine Verbindung der allgemeinen Formel (I) ist, die dadurch gekennzeichnet ist, daß $R^4$ für ein Wasserstoff- oder Halogenatom oder eine Hydroxy-, Nitro- oder $C_{1-3}$-Alkanoylgruppe steht.

8. Verfahren nach Anspruch 1, dadurch gekennzeichnet, daß man die Ausgangsmaterialien und die Reaktionsbedingungen so auswählt, daß das Produkt eine Verbindung der allgemeinen Formel (I) ist, die dadurch gekennzeichnet ist, daß $R^5$ für ein Wasserstoffatom oder eine $C_{1-6}$-Alkylgruppe oder eine $C_{2-4}$-Alkenylgruppe steht.

9. Verfahren nach Anspruch 1, dadurch gekennzeichnet, daß man die Ausgangsmaterialien und die Reaktionsbedingungen so auswählt, daß das Produkt eine Verbindung der allgemeinen Formel (I) ist, die dadurch gekennzeichnet ist, daß $R^4$ für ein Wasserstoffatom oder eine Hydroxylgruppe steht und $R^5$ für eine Wasserstoffatom steht.

10. Verfahren nach Anspruch 1, dadurch gekennzeichnet, daß man die Ausgangsmaterialien und die Reaktionsbedingungen so auswählt, daß das Produkt eine Verbindung der allgemeinen Formel (II):

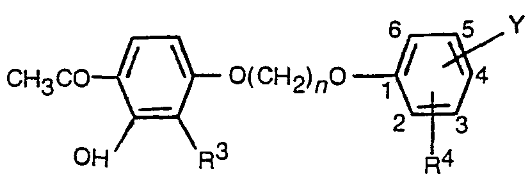

(II)

in der Y für eine Carbonsäuregruppe oder einen 5-1H-Tetrazolylring oder eine N-5-1H-Tetrazolylcarboxamidgruppe oder eine $C_{1-2}$-Alkyl-, $C_{1-2}$-Alkoxy- oder $C_2$-Alkenylkette, die endständig durch eine Carbonsäuregruppe substituiert ist und an der 3- oder 4-Stellung im Benzolring angeordnet ist, steht, n 1, 3 oder 5 ist und $R^3$ für eine $C_{3-6}$-Alkyl- oder $C_{3-6}$-Alkenylgruppe steht und $R^4$ für ein Wasserstoff- oder Bromatom oder eine Hydroxy-, Nitro- oder Acetylgruppe steht, oder ein physiologisch annehmbares Salz davon ist.

11. Verfahren nach Anspruch 1, dadurch gekennzeichnet, daß man die Ausgangsmaterialien und die Reaktionsbedingungen so auswählt, daß das Produkt eine Verbindung der allgemeinen Formel (II) ist, die dadurch gekennzeichnet ist daß n der Wert 3 hat.

12. Verfahren nach Anspruch 1, dadurch gekennzeichnet, daß man die Ausgangsmaterialien und die Reaktionsbedingungen so auswählt, daß das Produkt aus 4-[3-(4-Acetyl-3-hydroxy-2-propylphenoxy)-propoxy]-2-hydroxybenzoesäure, 4-[3-(4-Acetyl-3-hydroxy-2-propylphenoxy)-propoxy]-2-hydroxy-N-1H-tetrazol-5-y-benzamid, 3-[3-[3-(4-Acetyl-3-hydroxy-2-methylphenoxy)-propoxy]-phenyl]-2-propensäure und den physiologisch annehmbaren Salzen dieser Verbindungen ausgewählt ist.